# EUROPEAN PATENT SPECIFICATION

(11) **EP 3 455 223 B1**
(45) Date of publication and mention of the grant of the patent: **08.11.2023**
(21) Application number: 17796881.5
(22) Date of filing: 11.05.2017
(51) Int. Cl.: A61K 31/46, C07D 451/10, C07D 209/04, A61K 31/404, A61P 25/28, A61P 25/16, A61P 27/02

(54) **COMPOUNDS TO PROMOTE NORMAL PROCESSING OF APP**
VERBINDUNGEN ZUR FÖRDERUNG DER NORMALEN VERARBEITUNG VON APP
COMPOSÉS POUR FAVORISER LE TRAITEMENT NORMAL DE L'APP

(30) Priority: 12.05.2016 US 201662335533 P
(43) Date of publication of application: 20.03.2019
(73) Proprietor: Buck Institute for Research on Aging, Novato, CA 94945 (US)
(72) Inventor: JOHN, Varghese, San Francisco, CA 94122 (US); BREDESEN, Dale, E., Novato, CA 94949 (US); SPILMAN, Patricia, R., Mill Valley, CA 94941 (US); JAGODZINSKA, Barbara, Redwood City, CA 94061 (US)
(74) Representative: J A Kemp LLP
(86) International application number: PCT/US2017/032251
(87) International publication number: WO 2017/197177

(56) References cited:
- WO-A1-2013/123426
- WO-A1-2013/170147
- US-A- 4 789 673
- US-A1- 2014 364 451
- PAPKE, R. L. ET AL.: 'Molecular dissection of tropisetron, an a 7 nicotinic acetylcholine receptor-selective partial agonist' NEUROSCIENCE LETTERS vol. 378, no. 3, 2005, pages 140 - 144, XP027653569
- SPILMAN, P. ET AL.: 'The multi-functional drug tropisetron binds APP and normalizes cognition in a murine Alzheimer's model' BRAIN RESEARCH vol. 1551, 2014, pages 25 - 44, XP028614398

## Description

### BACKGROUND

Alzheimer's disease (AD) is a progressive neurodegenerative disorder that is characterized by rapid cognitive and functional decline in patients diagnosed with the disease. In the early stages of the disease the patients generally suffer from mild cognitive impairment (MCI) that converts over time to full blown AD. The disease broadly falls into two categories: a) late onset AD, which occurs generally at 65 years or older and is correlated to numerous risk factors including presence of an APOE ε4 allele; and b) early onset AD, which develops early on between 30 and 60 years of age and is generally associated with familial Alzheimer's disease (FAD) mutations in the amyloid precursor protein (APP) gene or in the presenilin gene. In both types of disease, the pathology is the same but the abnormalities tend to be more severe and widespread in cases beginning at an earlier age.

The disease is characterized by at least two types of lesions in the brain, senile plaques composed of the amyloid-β (Aβ) peptide (and other components, typically at lower concentrations than the Aβ peptide) and neurofibrillary tangles composed primarily of intracellular deposits of microtubule associated tau protein (especially hyperphosphorylated tau). Measurement of the levels of Aβ peptide and tau/phosphorylated tau (P-tau) in cerebrospinal fluid (CSF) along with imaging analysis and cognitive/functional tests are used clinically to determine progression of the disease and conversion to full-blown AD.

Alzheimer's disease (AD) has been viewed largely as a disease of toxicity, mediated by the collection of a small peptide (the Aβ peptide) that damages brain cells by physical and chemical properties, such as the binding of damaging metals, reactive oxygen species production, and direct damage to cell membranes. While such effects of Aβ have been clearly demonstrated, they do not offer a physiological role for the peptide.

Recent results from several different laboratories suggest Aβ has physiological signaling properties (*e.g.*, via interaction with APP itself, the insulin-receptor, and other receptors), and our results suggest that AD may result from an imbalance between two normal processes: memory formation and normal forgetting. Our studies show that APP has all of the characteristics of a dependence-receptor, *e*.*g*., a receptor that mediates cell-death in the presence of an anti-trophin (in this case, Aβ), but supports cell survival in the presence of a trophic-factor (such as laminin).

Several significant changes have occurred in the AD landscape recently. Two therapies showed marked reduction of Aβ levels in AD patients with limited to no cognitive improvement (Mangialasche, et al., (2010) Lancet Neurol. 9, 702-716*)*. This was unexpected by much of the research community, as AD has been largely viewed as a disease of chemical and physical toxicity of Aβ (*e*.*g*., generation of reactive oxygen species, metal binding, *etc*.). However, recent results from multiple laboratories suggest a completely different view of AD as an imbalance in physiological signal transduction.

For example Lu et al., (2000) Nat. Med., 6: 397-404 and Galvan et al., (2006) Proc. Natl. Acad. Sci. USA, 103:7130-7135 have shown a role of APP in mechanisms of signal transduction leading to neuronal cell death. These results, coupled with others, argue that Alzheimer's disease results from an imbalance between two normal processes: memory formation and normal forgetting. The data show that the Aβ peptide can play a role in modulating, processing and signaling through binding to the amyloid precursor protein (APP), and thus play a central role in the pathogenesis of Alzheimer's disease through signaling rather than chemical and physical effects.

APP695 can be cleaved by caspases at an intracellular site (Asp664), leading to the release of a small C31 peptide and an APPneo (APP664) fragment, and both products are potentially pro-apoptotic (Galavan *et al., supra*). Immunohistochemical analysis of AD brain demonstrates that this cytoplasmic cleavage occurs 4-fold more in AD brain than normal, and the products are found around plaques and tangles in key brain areas affected by the disease (Bredesen et al., (2006) Nature 443: 796-802). Through a single genetic mutation in the amyloid precursor protein (APP the AD phenotype was reversed in a transgenic mouse model by producing a mutation of aspartic acid residue 664 to alanine of APP695 leading to the complete blockage of the C-terminal cleavage *in vivo.* In addition, these transgenic mice demonstrate normal synaptic function and normal memory. Furthermore, it has been shown in cell culture that this C-terminal cleavage requires Aβ-facilitated APP multimerization.

The striking result of this research, indicates that molecules that increase sAPPα can normalize APP processing and improve behavioral symptoms associated with Alzheimer's disease. Furthermore, sAPPα has been shown to inhibit the β-secretase enzyme (Peters-Libeu et al. (2015) J. Alzheimer's Dis. 47: 545-555) and to modulate p-Tau levels (Dent et al. (2016) J. Neurochem., 135(3): 630-637) . This argues that this mechanism can be of fundamental importance in developing novel therapeutic agents for treatment for AD.

US 4789673 describes that dicarboxylic heterocyclic and substituted benzoic acid alkylene bridged piperidyl amides and esters are serotonin M antagonists. US2014/364451 describes tropinol esters and related compounds to promote normal processing of APP. WO2013/123426 describes formulations and methods for the treatment or prophylaxis of pre-MCI and/or pre-Alzheimer's conditions.

### SUMMARY

Various embodiments contemplated herein may include, but need not be limited to, one or more of the following:
Embodiment 1: A compound according to the formula 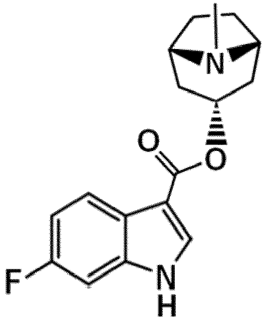 or an enantiomer, a mixture of enantiomers, or a mixture of two or more diastereomers thereof; or a pharmaceutically acceptable salt, solvate or hydrate thereof
Embodiment 2: The compound of embodiment 1, wherein said compound comprises a pharmaceutically acceptable salt.
Embodiment 3: The compound of embodiment 2, wherein said pharmaceutically acceptable salt is a salt of an acid selected from the group consisting of 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid (D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (- L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid (- L), salicylic acid, sebacic acid, stearic acid, succinic acid, sulfuric acid, tartaric acid (+ L), thiocyanic acid, toluenesulfonic acid (p), acid undecylenic acid.
Embodiment 4: The compound of embodiment 2, wherein said pharmaceutically acceptable salt is HCl.
Embodiment 5: A pharmaceutical formulation comprising the compound according to any one of embodiments 1-4, and a pharmaceutically acceptable carrier or excipient.
Embodiment 6: The formulation of embodiment 5, wherein said formulation is formulated for administration via a route selected from the group consisting of oral administration, nasal administration, administration via inhalation, oral administration, rectal administration, intraperitoneal injection, intravascular injection, subcutaneous injection, transcutaneous administration, and intramuscular injection.
Embodiment 7: The formulation according to any one of embodiments 5-6, wherein said formulation is a unit dosage formulation.
Embodiment 8: The formulation according to any one of embodiments 5-7, wherein said formulation is sterile.
Embodiment 9: A compound according to any one of embodiments 1-4, or a pharmaceutical formulation according to any one of embodiments 5-8, for use in a method of mitigating in a mammal one or more symptoms associated with a disease characterized by amyloid deposits in the brain, or delaying or preventing the onset of said symptoms, said method comprising:
   administering, or causing to be administered, to said mammal a compound according to any one of embodiments 1-4, or a pharmaceutical formulation according to any one of embodiments 5-8, wherein said administering is in an amount sufficient to mitigate said one or more symptoms.
Embodiment 10: A compound according to any one of embodiments 1-4, or a pharmaceutical formulation according to any one of embodiments 5-8, for use in a method of reducing the risk, lessening the severity, or delaying the progression or onset of a disease characterized by beta-amyloid deposits in the brain of a mammal, said method comprising:
   administering, or causing to be administered, to said mammal a compound according to any one of embodiments 1-4, or a pharmaceutical formulation according to any one of embodiments 5-8, wherein said administering is in an amount sufficient to reducing the risk, lessen the severity, or delay the progression or onset of said disease.
Embodiment 11: The compound or formulation for use according to embodiments 9- 10, wherein said disease is a disease selected from the group consisting of Alzheimer's disease, Cerebrovascular dementia, Parkinson's disease, Huntington's disease, Cerebral amyloid angiopathy, amytrophic lateral sclerosis (ALS), traumatic brain injury (TBI), and stroke.
Embodiment 12: A compound according to any one of claims 1-4, or a pharmaceutical formulation according to any one of claims 5-8, for use in a method of preventing or delaying the onset of a pre-Alzheimer's condition and/or cognitive dysfunction, and/or ameliorating one or more symptoms of a pre-Alzheimer's condition and/or cognitive dysfunction, or preventing or delaying the progression of a pre-Alzheimer's condition or cognitive dysfunction to Alzheimer's disease in a mammal, said method comprising:
   administering, or causing to be administered, to said mammal a compound according to any one of embodiments 1-4, or a pharmaceutical formulation according to any one of embodiments 5-8, wherein said administering is in an amount sufficient to promote the processing of amyloid precursor protein (APP) by the non-amyloidogenic pathway.
Embodiment 13: The compound or formulation for use according to any one of embodiments 9 to 12, wherein administration of said compound delays or prevents the progression of MCI to Alzheimer's disease.
Embodiment 14: The compound or formulation for use according to any one of embodiments 9-11, wherein the disease is Alzheimer's disease.
Embodiment 15: The compound or formulation for use according to any one of embodiments 9 to 14, wherein the mammal is at risk of developing Alzheimer's disease.
Embodiment 16: The compound or formulation for use according to embodiment 15, wherein the mammal has a familial risk for having Alzheimer's disease, and/or the mammal has a familial Alzheimer's disease (FAD) mutation, and/or the mammal has the APOE ε4 allele.
Embodiment 17: A kit comprising: a container containing a compound according to any one of embodiments 1-4, or a pharmaceutical formulation according to any one of embodiments 5-8; and instructional materials teaching the use of said composition to mitigate one or more symptoms associated with a disease characterized by amyloid deposits in the brain, and/or the use of said composition in delaying or preventing the onset of one or more of said symptoms.

### DEFINITIONS

Generally, reference to a certain element such as hydrogen or H is meant to include all isotopes of that element. For example, if an R group is defined to include hydrogen or H, it also includes deuterium and tritium. Accordingly, isotopically labeled compounds are within the scope of this invention.

As used herein, the phrase "a subject in need thereof" refers to a subject, as described *infra,* that suffers or is at a risk of suffering (*e.g.*, pre-disposed such as genetically pre-disposed) from the diseases or conditions listed herein.

The terms "subject," "individual," and "patient" may be used interchangeably and refer to a mammal, preferably a human or a non-human primate, but also domesticated mammals (*e.g*., canine or feline), laboratory mammals (*e.g.*, mouse, rat, rabbit, hamster, guinea pig), and agricultural mammals (*e.g.*, equine, bovine, porcine, ovine). In various embodiments, the subject can be a human (*e.g.*, adult male, adult female, adolescent male, adolescent female, male child, female child) under the care of a physician or other health worker in a hospital, psychiatric care facility, as an outpatient, or other clinical context. In certain embodiments, the subject may not be under the care or prescription of a physician or other health worker.

An "effective amount" refers to an amount effective, at dosages and for periods of time necessary, to achieve the desired therapeutic or prophylactic result. A "therapeutically effective amount" of a multi-component formulation, optionally in combination with one or more pharmaceuticals, may vary according to factors such as the disease state, age, sex, and weight of the individual, the pharmaceutical (and dose thereof) when used in combination with pharmaceutical, and the ability of the treatment to elicit a desired response in the individual. A therapeutically effective amount is also one in which any toxic or detrimental effects of a treatment are substantially absent or are outweighed by the therapeutically beneficial effects. The term "therapeutically effective amount" refers to an amount of an active agent or composition comprising the same that is effective to "treat" a disease or disorder in a mammal (*e.g.*, a patient). In one embodiment, a therapeutically effective amount is an amount sufficient to improve at least one symptom associated with a neurological disorder, improve neurological function, improve cognition, or one or more markers of a neurological disease, or to enhance the efficacy of one or more pharmaceuticals administered for the treatment or prophylaxis of a neurodegenerative pathology. In certain embodiments, an effective amount is an amount sufficient alone, or in combination with a pharmaceutical agent to prevent advancement or the disease, delay progression, or to cause regression of a disease, or which is capable of reducing symptoms caused by the disease,

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount is less than the therapeutically effective amount.

The terms "treatment," "treating," or "treat" as used herein, refer to actions that produce a desirable effect on the symptoms or pathology of a disease or condition, particularly those that can be effected utilizing the multi-component formulation(s) described herein, and may include, but are not limited to, even minimal changes or improvements in one or more measurable markers of the disease or condition being treated. Treatments also refers to delaying the onset of, retarding or reversing the progress of, reducing the severity of, or alleviating or preventing either the disease or condition to which the term applies, or one or more symptoms of such disease or condition. "Treatment," "treating," or "treat" does not necessarily indicate complete eradication or cure of the disease or condition, or associated symptoms thereof. In one embodiment, treatment comprises improvement of at least one symptom of a disease being treated. The improvement may be partial or complete. The subject receiving this treatment is any subject in need thereof. Exemplary markers of clinical improvement will be apparent to persons skilled in the art.

The term "mitigating" refers to reduction or elimination of one or more symptoms of that pathology or disease, and/or a reduction in the rate or delay of onset or severity of one or more symptoms of that pathology or disease, and/or the prevention of that pathology or disease.

As used herein, the phrases "improve at least one symptom" or "improve one or more symptoms" or equivalents thereof, refer to the reduction, elimination, or prevention of one or more symptoms of pathology or disease. Illustrative symptoms of pathologies treated, ameliorated, or prevented by the compositions described herein (e.g., compound C41, or an enantiomer, a mixture of enantiomers, or a mixture of two or more diastereomers thereof, or a pharmaceutically acceptable salt, ester, amide, solvate or hydrate thereof, or derivatives thereof) include, but are not limited to, reduction, elimination, or prevention of one or more markers that are characteristic of the pathology or disease (*e.g.*, of total Tau (tTau), phospho-Tau (pTau), APPneo, soluble Aβ40, Aβ, pTau/Aβ40 ratio and tTau/Aβ42 ratio, and/or an increase in the CSF of levels of one or more components selected from the group consisting of Aβ42/Aβ40 ratio, Aβ42/Aβ38 ratio, sAPPα, sAPPβ, sAPPα/sAPPβ ratio, sAPPα/Aβ40 ratio, sAPPα/Aβ42 ratio, *etc*.) and/or reduction, stabilization or reversal of one or more diagnostic criteria (*e.g.*, clinical dementia rating (CDR)). Illustrative measures for improved neurological function include, but are not limited to the use of the mini-mental state examination (MMSE) or Folstein test (a questionnaire test that is used to screen for cognitive impairment), the General Practitioner Assessment of Cognition (GPCOG), a brief screening test for cognitive impairment described by Brodaty et al., (2002) Geriatrics Society 50(3): 530-534, and the like.

The phrase "cause to be administered" refers to the actions taken by a medical professional (*e.g*., a physician), or a person prescribing and/or controlling medical care of a subject, that control and/or determine, and/or permit the administration of the agent(s)/compound(s) at issue to the subject. Causing to be administered can involve diagnosis and/or determination of an appropriate therapeutic or prophylactic regimen, and/or prescribing particular agent(s)/compounds for a subject. Such prescribing can include, for example, drafting a prescription form, annotating a medical record, and the like.

### BRIEF DESCRIPTION OF THE DRAWINGS

Figure 1 illustrates the structure of compound C41.
Figure 2 illustrates the Synthesis Scheme 1 for the synthesis of compound C41. Reagents and conditions: (a) i. DMF, 0°C, 3h ii. 4M NaOH, reflux, 4h ; (b) oxalyl chloride, DCM, tropine, overnight; (c) 4M HCl, 1,4-dioxan, 2h.
Figure 3 illustrates the structure of tropisetron (Navoban).
Figure 4 illustrates the results of a primary screen of compound C41 (C41 HCl) for effects on sAPPα in SH-SY5Y cells.
Figure 5 illustrates the results of the secondary screen of compound C41 (C41 HCl) for effects on sAPPα, Aβ42, and the sAPPα/ Aβ42 ratio in CHO-7W cells.
Figure 6 illustrates the results of pharmacokinetics for F03 (upper panel) as compared to C41 (lower panel). Left graphs show results for both subcutaneous injection and oral delivery, right graphs show compound levels after oral delivery only.
Figure 7, panels A-C, illustrates the results of an *in vivo* study of C41 and F03 in an AD model mouse. Panel A: Novel object preference; Panel B: Novel location preference; Panel C: sAPPα.
Figure 8 panels A-B, illustrates the results of an *in vivo* study of C41 and F03 in an AD model mouse. Panel A: sAPPβ; Panel B: the sAPPα/sAPPβ ratio.
Figure 9, panels A-C, illustrates the results of an *in vivo* study of C41 and F03 in an AD model mouse. Panel A: Aβ42; Panel B: Aβ40/42 ratio; Panel C: sAPPα/Aβ42 ratio.
Figure 10 illustrates effects of the first batch (C41(1)) and the second batch (C41(2)) of C41 and F03 on sAPPα in SH-SY5Y cells.
Figure 11 illustrates the Perkin-Elmer AlphaLISA assay used for sAPPα, sAPPβ, Tau, and p-Tau determination.
Figure 12 shows C41 pharmacokinetics in rats. Top panel: Levels after oral delivery at 10 mk at 1, 8, and 24 hours; levels after oral delivery at 40 mk at 24 hours only. Bottom panel: IV delivery at 2.5 mk. Note brain levels in ng/g, plasma levels in ng/ml.
Figure 13 illustrates C41 pharmacokinetic parameters in rat. The graph shows area under the curve for plasma levels after oral delivery as compared to iv delivery, adjusted for dose.
Figure 14 illustrates subchronic non-GLP toxicity of C41 after oral gavage in rats for 14 days, done at Absorption Systems. The dose was 40 mg/kg/day via oral gavage. The animals showed no weight loss and no adverse effects. The rats were adult Sprague-Dawley rats (6 per group, two groups (C41 and Vehicle only)). Blood collection was at 1, 5, 15, and 30 minutes, and at 1, 3, 8, and 24 hours on days 1 and 14. Tissue - Brain and 12 other organs were collected and the end of the study. Brain levels are shown as ng/g.
Figure 15 illustrates the result of C41 lead confirmation in B254 mice. Panel A: Novel Location Recognition; Panel B: Novel Object Recognition; Panel C: sAPPα; Panel D: Aβ40; Panel E: Aβ42; Panel F: p-tau/tau.

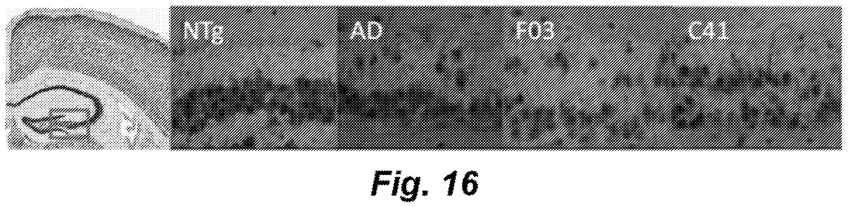

Figure 16 illustrates mechanistic confirmation studies for determination of synaptic load. C41 and tropisetron (F03) similarly restore synaptic density as reflected by green fluorescent synaptophysin labeling of hippocampus in an AD mouse model.

### DETAILED DESCRIPTION

The prevailing view of Alzheimer's disease (AD) is that amyloid-beta peptides cause toxicity through chemical and physical mechanisms, such as metal binding, ROS production, and membrane damage. Our data suggest an alternative view of AD as an imbalance in physiological signaling mediated by APP. In this model, Aβ functions physiologically as an anti-trophin, and Aβ binding to APP induces the formation of peptides that mediate neurite retraction and cell death (*see, e.g.,* Lu et al., (2000) Nat. Med., 6: 397-404).

The references to methods of treatment in the detailed description of the invention in this description are to be interpreted as references to the compounds, pharmaceutical compositions and medicaments of the present invention for use in a method for treatment of the human (or animal) body by therapy (or for diagnosis).

In various embodiments compositions and methods are provided for mitigating in a mammal one or more symptoms associated with a disease characterized by amyloid deposits in the brain (*e.g.*, Alzheimer's disease, Cerebrovascular dementia, Parkinson's disease, Huntington's disease, Cerebral amyloid angiopathy, *etc.),* or delaying or preventing the onset of symptoms. Compositions and methods are also provided for reducing the risk, lessening the severity, or delaying the progression or onset of a disease characterized by beta-amyloid deposits in the brain of a mammal (*e.g.*, Alzheimer's disease, Cerebrovascular dementia, Parkinson's disease, Huntington's disease, Cerebral amyloid angiopathy, *etc*.). In certain embodiments compositions and methods are provided for preventing or delaying the onset of a pre-Alzheimer's condition and/or cognitive dysfunction, and/or ameliorating one or more symptoms of a pre-Alzheimer's condition and/or cognitive dysfunction, or preventing or delaying the progression of a pre-Alzheimer's condition or cognitive dysfunction to Alzheimer's disease in a mammal. In certain embodiments compositions and methods are provided for promoting the processing of amyloid precursor protein (APP) by the non-amyloidogenic pathway as characterized by increasing sAPPα and/or the sAPPα/Aβ42 ratio in a mammal. In certain embodiments compositions and methods are provided for inhibiting the C-terminal cleavage of APP resulting in the formation of APP-C31 peptide and APPneo (APP664) in a mammal.

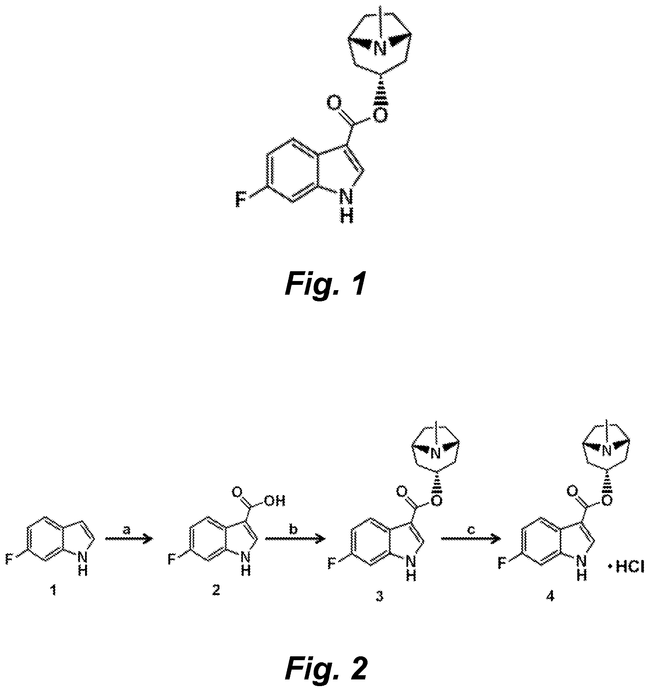

The methods described herein are based, in part, on the surprising discovery that the compound C41 (*see,* Fig. 1, or an enantiomer, a mixture of enantiomers, or a mixture of two or more diastereomers thereof, or a pharmaceutically acceptable salt, ester, amide, solvate or hydrate thereof, or derivatives thereof) shows enhanced transport across the blood brain barrier, and or enhanced bioavailability (amount, persistence, stability, *etc*.) and, accordingly enhanced efficacy in promoting processing of amyloid beta (Aβ) precursor protein ("APP") by the nonamyloidogenic ("anti-AD") pathway and/or in reducing or inhibiting processing of APP by the amyloidogenic ("pro-AD") pathway as compared for example to the corresponding amide forms. Without being bound to a particular theory, this is believed to result in reduced production of Aβ that may be deposited in amyloid plaques in the brain and the other pro-AD fragments known to result in neurotoxicity.

Accordingly, in various embodiments, the use of compound C41 (*see,* Fig. 1) or formulations thereof and/or an enantiomer, a mixture of enantiomers, or a mixture of two or more diastereomers thereof; or a pharmaceutically acceptable salt, ester, amide, solvate or hydrate thereof, or a derivative thereof for the modulation, and in particular in the reduction of amyloidogenic pathologies (*e.g.*, Alzheimer's disease, age-related macular degeneration (AMD), Cerebrovascular dementia, Parkinson's disease, and the like) is provided. In certain embodiments, the C41 compounds or formulations thereof are used to prevent or delay the onset of a pre-Alzheimer's condition and/or cognitive dysfunction, and/or to ameliorate one or more symptoms of a pre-Alzheimer's condition and/or cognitive dysfunction, and/or to prevent or delay the progression of a pre-Alzheimer's condition or cognitive dysfunction to Alzheimer's disease. In certain embodiments, the C41 compounds and formulations thereof are used in a method of mitigating in a mammal one or more symptoms associated with a disease characterized by amyloid deposits in the brain, or delaying or preventing the onset of said symptoms. In certain embodiments, methods of reducing the risk, lessening the severity, or delaying the progression or onset of a disease characterized by beta-amyloid deposits in the brain of a mammal are also provided. In addition, methods of promoting the processing of amyloid precursor protein (APP) by the non-amyloidogenic pathway in a mammal are provided. In certain embodiments, methods of directly or indirectly inhibiting the C-terminal cleavage of APP resulting in the formation of APP-C31 peptide and APPneo (APP₆₆₄) in a mammal are provided.

Typically each of these methods involve administering a C41 compound or formulations thereof and/or an enantiomer, a mixture of enantiomers, or a mixture of two or more diastereomers thereof; or a pharmaceutically acceptable salt, ester, amide, solvate or hydrate thereof, or a derivative thereof, in an amount sufficient to produce the desired activity (*e.g*., mitigating one or more symptoms associated with a disease characterized by amyloid deposits in the brain, or delaying or preventing the onset of said symptoms, and/or reducing the risk, lessening the severity, or delaying the progression or onset of a disease characterized by beta-amyloid deposits in the brain of a mammal, and/or promoting the processing of amyloid precursor protein (APP) by the non-amyloidogenic pathway).

While the methods described herein are detailed primarily in the context of mild cognitive impairment (MCI) and Alzheimer's disease (AD) it is believed they can apply equally to other pathologies characterized by amyloidosis. Illustrative, but non-limiting list of conditions characterized by amyloid plaque formation are shown in Table 1.

**Table 1. Illustrative, but non-limiting pathologies characterized by amyloid formation/deposition.**

| **Disease** | **Characteristic Protein** | **Abbreviation** |
|---|---|---|
| Alzheimer's disease | Beta amyloid | Aβ |
| Diabetes mellitus type 2 | IAPP (Amylin) | AIAPP |
| Parkinson's disease | Alpha-synuclein | |
| Transmissible spongiform encephalopathy *e.g*. Bovine spongiform encephalopathy | Prion | APrP |
| Huntington's Disease | Huntingtin | |
| Medullary carcinoma of the thyroid | Calcitonin | ACal |
| Cardiac arrhythmias, Isolated atrial amyloidosis | Atrial natriuretic factor | AANF |
| Atherosclerosis | Apolipoprotein AI | AApoA1 |
| Rheumatoid arthritis | Serum amyloid A | AA |
| Aortic medial amyloid | Medin | AMed |
| Prolactinomas | Prolactin | APro |
| Familial amyloid polyneuropathy | Transthyretin | ATTR |
| Hereditary non-neuropathic systemic amyloidosis | Lysozyme | ALys |
| Dialysis related amyloidosis | Beta 2 microglobulin | Aβ2M |
| Finnish amyloidosis | Gelsolin | AGel |
| Lattice corneal dystrophy | Keratoepithelin | AKer |
| Cerebral amyloid angiopathy | Beta amyloid^{[15]} | Aβ |
| Cerebral amyloid angiopathy (Icelandic type) | Cystatin | ACys |
| systemic AL amyloidosis | Immunoglobulin light chain AL^{[14]} | AL |
| Sporadic Inclusion Body Myositis | S-IBM | none |
| Age-related macular degeneration (AMD) | | |
| Cerebrovascular dementia | | |

### Subjects Who Can Benefit from the Present Methods

Subjects/patients amenable to treatment using the methods described herein include individuals at risk of disease (*e.g.*, a pathology characterized by amyloid plaque formation) but not showing symptoms, as well as subjects presently showing symptoms. Accordingly, certain subjects include subjects at increased risk for the onset of a pre-Alzheimer's condition and/or cognitive dysfunction (*e.g.*, MCI), and/or subjects diagnosed as having a pre-Alzheimer's condition and/or cognitive dysfunction (*e.g.*, MCI).

Accordingly, in various embodiments, therapeutic and/or prophylactic methods are provided that utilize the active agent(s) (*e.g.,* a C41 compound described herein, or formulations thereof and/or an enantiomer, a mixture of enantiomers, or a mixture of two or more diastereomers thereof; or a pharmaceutically acceptable salt, ester, amide, solvate or hydrate thereof, or a derivative thereof) are provided. Typically the methods involve administering one or more active agent(s) (*e.g.*, a C41 compound) to a subject (*e.g.*, to a human in need thereof) in an amount sufficient to realize the desired therapeutic or prophylactic result.

### Prophylaxis

In certain embodiments, active agent(s) (*e.g.*, a C41 compound or formulation, and/or an enantiomer, a mixture of enantiomers, or a mixture of two or more diastereomers thereof; or a pharmaceutically acceptable salt, ester, amide, solvate or hydrate thereof or derivatives thereof) are utilized in various prophylactic contexts. Thus, for example, in certain embodiments, the active agent(s) (*e.g.,* a C41 compound and/or formulation thereof) can be used to prevent or delay the onset of a pre-Alzheimer's cognitive dysfunction, and/or to ameliorate one more symptoms of a pre-Alzheimer's condition and/or cognitive dysfunction, and/or to prevent or delay the progression of a pre-Alzheimer's condition and/or cognitive dysfunction to Alzheimer's disease.

Accordingly in certain embodiments, the prophylactic methods described herein are contemplated for subjects identified as "at risk" and/or as having evidence of early Alzheimer's Disease (AD) pathological changes, but who do not meet clinical criteria for MCI or dementia. Without being bound to a particular theory, it is believed that even this "preclinical" stage of the disease represents a continuum from completely asymptomatic individuals with biomarker evidence suggestive of AD-pathophysiological process(es) (abbreviated as AD-P, *see, e.g.,* Sperling et al., (2011) Alzheimer's & Dementia, 1-13) at risk for progression to AD dementia to biomarker-positive individuals who are already demonstrating very subtle decline but not yet meeting standardized criteria for MCI (*see, e.g.,* Albert et al., (2011) Alzheimer's and Dementia, 1-10 (doi:10.1016/j.jalz.2011.03.008)).

This latter group of individuals might be classified as "not normal, not MCI" but can be designated "pre-symptomatic" or "pre-clinical or "asymptomatic" or "pre-manifest"). In *various* embodiments, this continuum of pre-symptomatic AD can also encompass (1) individuals who carry one or more apolipoprotein E (APOE) ε4 alleles who are known or believed to have an increased risk of developing AD dementia, at the point they are AD-P biomarker-positive, and (2) carriers of autosomal dominant mutations, who are in the pre-symptomatic biomarker-positive stage of their illness, and who will almost certainly manifest clinical symptoms and progress to dementia.

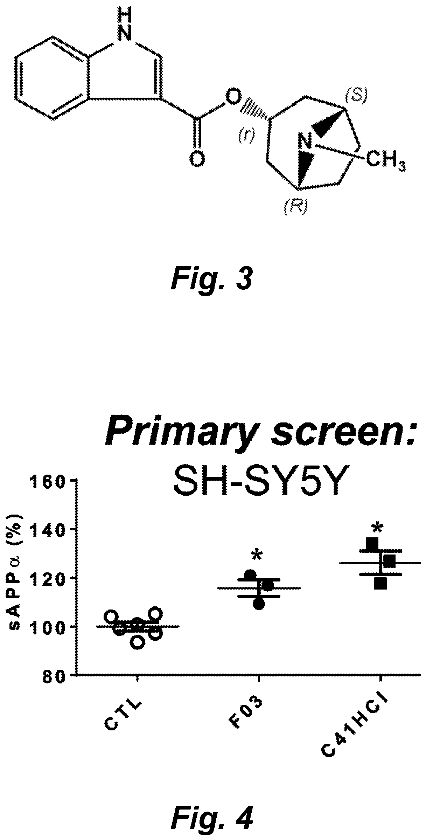

A biomarker model has been proposed in which the most widely validated biomarkers of AD-P become abnormal and likewise reach a ceiling in an ordered manner (*see, e.g.,* Jack et al., (2010) Lancet Neurol., 9: 119-128.). This biomarker model parallels proposed pathophysiological sequence of (pre-AD/AD), and is relevant to tracking the preclinical (asymptomatic) stages of AD (*see, e.g.,* Figure 3 in Sperling et al., (2011) Alzheimer's & Dementia, 1-13). Biomarkers of brain amyloidosis include, but are not limited to reductions in CSF Aβ42 and increased amyloid tracer retention on positron emission tomography (PET) imaging. Elevated CSF tau is not specific to AD and is thought to be a biomarker of neuronal injury. Decreased fluorodeoxyglucose 18F (FDG) uptake on PET with a temporoparietal pattern of hypometabolism is a biomarker of AD-related synaptic dysfunction. Brain atrophy on structural magnetic resonance imaging (MRI) in a characteristic pattern involving the medial temporal lobes, paralimbic and temporoparietal cortices is a biomarker of AD-related neurodegeneration. Other markers include, but are not limited to volumetric MRI, FDG-PET, or plasma biomarkers (*see, e.g.,* Vemuri et al., (2009) Neurology, 73: 294-301; Yaffe et al., (2011) JAMA 305: 261-266).

In certain embodiments, the subjects suitable for the prophylactic methods contemplated herein include, but are not limited to subject characterized as having asymptomatic cerebral amyloidosis. In *various* embodiments, these individuals have biomarker evidence of Aβ accumulation with elevated tracer retention on PET amyloid imaging and/or low Aβ42 in CSF assay, but typically no detectable evidence of additional brain alterations suggestive of neurodegeneration or subtle cognitive and/or behavioral symptomatology.

It is noted that currently available CSF and PET imaging biomarkers of Aβ primarily provide evidence of amyloid accumulation and deposition of fibrillar forms of amyloid. Data suggest that soluble or oligomeric forms of Aβ are likely in equilibrium with plaques, which may serve as reservoirs. In certain embodiments, it is contemplated that there is an identifiable preplaque stage in which only soluble forms of Aβ are present. In certain embodiments, it is contemplated that oligomeric forms of amyloid may be critical in the pathological cascade, and provide useful markers. In addition, early synaptic changes may be present before evidence of amyloid accumulation.

In certain embodiments, the subjects suitable for the prophylactic methods contemplated herein include, but are not limited to, subjects characterized as amyloid positive with evidence of synaptic dysfunction and/or early neurodegeneration. In *various* embodiments, these subjects have evidence of amyloid positivity and presence of one or more markers of "downstream" AD-P-related neuronal injury. Illustrative, but non-limiting markers of neuronal injury include, but are not limited to (1) elevated CSF tau or phospho-tau, (2) hypometabolism in an AD-like pattern (*e.g.*, posterior cingulate, precuneus, and/or temporoparietal cortices) on FDG-PET, and (3) cortical thinning/gray matter loss in a specific anatomic distribution (*e.g.*, lateral and medial parietal, posterior cingulate, and lateral temporal cortices) and/or hippocampal atrophy on volumetric MRI. Other markers include, but are not limited to fMRI measures of default network connectivity. In certain embodiments, early synaptic dysfunction, as assessed by functional imaging techniques such as FDG-PET and fMRI, can be detectable before volumetric loss. Without being bound to a particular theory, it is believed that amyloid-positive individuals with evidence of early neurodegeneration may be farther down the trajectory (*e.g.*, in later stages of preclinical (asymptomatic) AD).

In certain embodiments, the subjects suitable for the prophylactic methods contemplated herein include, but are not limited to, subjects characterized as amyloid positive with evidence of neurodegeneration and subtle cognitive decline. Without being bound to a particular theory, it is believed that those individuals with biomarker evidence of amyloid accumulation, early neurodegeneration, and evidence of subtle cognitive decline are in the last stage of preclinical (asymptomatic) AD, and are approaching the border zone with clinical criteria for mild cognitive impairment (MCI). These individuals may demonstrate evidence of decline from their own baseline (particularly if proxies of cognitive reserve are taken into consideration), even if they still perform within the "normal" range on standard cognitive measures. Without being bound to a particular theory, it is believed that more sensitive cognitive measures, particularly with challenging episodic memory measures, may detect very subtle cognitive impairment in amyloid-positive individuals. In certain embodiments, criteria include, but are not limited to, self-complaint of memory decline or other subtle neurobehavioral changes.

As indicated above, subjects/patients amenable to prophylactic methods described herein include individuals at risk of disease (*e.g*., a pathology characterized by amyloid plaque formation such as MCI) but not showing symptoms, as well as subjects presently showing certain symptoms or markers. It is known that the risk of MCI and later Alzheimer's disease generally increases with age. Accordingly, in asymptomatic subjects with no other known risk factors, in certain embodiments, prophylactic application is contemplated for subjects over 50 years of age, or subjects over 55 years of age, or subjects over 60 years of age, or subjects over 65 years of age, or subjects over 70 years of age, or subjects over 75 years of age, or subjects over 80 years of age, in particular to prevent or slow the onset or ultimate severity of mild cognitive impairment (MCI), and/or to slow or prevent the progression from MCI to early stage Alzheimer's disease (AD).

In certain embodiments, the methods described herein present methods are especially useful for individuals who do have a known genetic risk of Alzheimer's disease (or other amyloidogenic pathologies), whether they are asymptomatic or showing symptoms of disease. Such individuals include those having relatives who have experienced MCI or AD (*e.g*., a parent, a grandparent, a sibling), and those whose risk is determined by analysis of genetic or biochemical markers. Genetic markers of risk toward Alzheimer's disease include, for example, mutations in the APP gene, particularly mutations at position 717 and positions 670 and 671 referred to as the Hardy and Swedish mutations respectively (*see*, *e.g.,* Hardy (1997) Trends. Neurosci., 20: 154-159). Other markers of risk include mutations in the presenilin genes (PS1 and PS2), family history of AD, having the familial Alzheimer's disease (FAD) mutation, the APOE ε4 allele, hypercholesterolemia or atherosclerosis. Further susceptibility genes for the development of Alzheimer's disease are reviewed, *e.g.,* in Sleegers, et al., (2010) Trends Genet. 26(2): 84-93.

In some embodiments, the subject is asymptomatic but has familial and/or genetic risk factors for developing MCI or Alzheimer's disease. In asymptomatic patients, treatment can begin at any age (*e.g*., 20, 30, 40, 50, years of age). Usually, however, it is not necessary to begin treatment until a patient reaches at least about 40, 50, 60, 70 or 80 years of age.

In some embodiments, the subject is one who exhibits symptoms, for example, of mild cognitive impairment (MCI) or Alzheimer's disease (AD). Individuals presently suffering from Alzheimer's disease can be recognized from characteristic dementia, as well as the presence of risk factors described above. In addition, a number of diagnostic tests are available for identifying individuals who have AD. These include, but are not limited to measurement of CSF Tau, phospho-tau (pTau), Aβ42 levels and C-terminally cleaved APP fragment (APPneo). Elevated total-Tau (tTau), phospho-Tau (pTau), APPneo, soluble Aβ40, pTau/Aβ42 ratio and tTau/Aβ42 ratio, and decreased Aβ42 levels, Aβ42/Aβ40 ratio, Aβ42/Aβ38 ratio, sAPPα levels, sAPPα/sAPPβ ratio, sAPPα/Aβ40 ratio, and sAPPα/Aβ42 ratio signify the presence of AD. In some embodiments, the subject or patient is clinically diagnosed as having MCI. Increased levels of neural thread protein (NTP) in urine and/or increased levels of α2-macroglobulin (α2M) and/or complement factor H (CFH) in plasma are also biomarkers of MCI and/or AD (*see, e.g.,* Anoop et al., (2010) Int. J. Alzheimer's Dis.2010:606802).

In certain embodiments, subjects amenable to treatment may have ageassociated memory impairment (AAMI), or mild cognitive impairment (MCI). The methods described herein are particularly well-suited to the prophylaxis and/or treatment of MCI, particularly MCI characterized by an amyloidogenic process. In such instances, the methods can delay or prevent the onset of MCI, and or reduce one or more symptoms characteristic of MCI and/or delay or prevent the progression from MCI to early-, mid- or late- stage Alzheimer's disease, and/or reduce the ultimate severity of the disease.

### Mild Cognitive Impairment (MCI)

In various embodiments, the C41 compounds (*e.g*., or formulations therof, and/or an enantiomer, a mixture of enantiomers, or a mixture of two or more diastereomers thereof; or a pharmaceutically acceptable salt, ester, amide, solvate or hydrate thereof or derivatives thereof) can be used for the treatment and/or prophylaxis of age-related cognitive decline and/or for the treatment and/or prophylaxis of mild cognitive impairment (MCI). Mild cognitive impairment, also known as incipient dementia, or isolated memory impairment) is a diagnosis given to individuals who have cognitive impairments beyond that expected for their age and education, but that typically do not interfere significantly with their daily activities (*see, e.g.,* Petersen et al., (1999) Arch. Neurol. 56(3): 303-308). It is considered in many instances to be a boundary or transitional stage between normal aging and dementia. Although MCI can present with a variety of symptoms, when memory loss is the predominant symptom it is termed "amnestic MCI" and is can be a risk factor for Alzheimer's disease (see, *e.g*., Grundman et al., (2004) Arch. Neurol. 61(1): 59-66; and on the internet at en.wikipedia.org/wiki/Mild_cognitive_impairment - cite_note-Grundman-1). When individuals have impairments in domains other than memory it is often classified as non-amnestic single- or multiple-domain MCI and these individuals are believed to be more likely to convert to other dementias (*e.g.* dementia with Lewy bodies). There is evidence suggesting that while amnestic MCI patients may not meet neuropathologic criteria for Alzheimer's disease, patients may be in a transitional stage of evolving Alzheimer's disease; patients in this hypothesized transitional stage demonstrated diffuse amyloid in the neocortex and frequent neurofibrillary tangles in the medial temporal lobe (see, *e.g.*, Petersen et al., (2006) Arch. Neurol., 63(5): 665-72).

The diagnosis of MCI typically involves a comprehensive clinical assessment including clinical observation, neuroimaging, blood tests and neuropsychological testing. In certain embodiments, diagnostic criteria for MCI include, but are not limited to those described by Albert et al., (2011) Alzheimer's & Dementia. 1-10. As described therein, diagnostic criteria include (1) core clinical criteria that could be used by healthcare providers without access to advanced imaging techniques or cerebrospinal fluid analysis, and (2) research criteria that could be used in clinical research settings, including clinical trials. The second set of criteria incorporate the use of biomarkers based on imaging and cerebrospinal fluid measures. The final set of criteria for mild cognitive impairment due to AD has four levels of certainty, depending on the presence and nature of the biomarker findings.

In certain embodiments, clinical evaluation/diagnosis of MCI involves: (1) Concern reflecting a change in cognition reported by patient or informant or clinician (*e.g*., historical or observed evidence of decline over time); (2) Objective evidence of Impairment in one or more cognitive domains, typically including memory (*e.g*., formal or bedside testing to establish level of cognitive function in multiple domains); (3) Preservation of independence in functional abilities; (4) Not demented; and in certain embodiments, (5) An etiology of MCI consistent with AD pathophysiological processes. Typically vascular, traumatic, medical causes of cognitive decline are ruled out where possible. In certain embodiments, evidence of longitudinal decline in cognition is identified, when feasible. Diagnosis is reinforced by a history consistent with AD genetic factors, where relevant.

With respect to impairment in cognitive domain(s), there should be evidence of concern about a change in cognition, in comparison with the person's previous level. There should be evidence of lower performance in one or more cognitive domains that is greater than would be expected for the patient's age and educational background. If repeated assessments are available, then a decline in performance should be evident over time. This change can occur in a variety of cognitive domains, including memory, executive function, attention, language, and visuospatial skills. An impairment in episodic memory (*e.g*., the ability to learn and retain new information) is seen most commonly in MCI patients who subsequently progress to a diagnosis of AD dementia.

With respect to preservation of independence in functional abilities, it is noted that persons with MCI commonly have mild problems performing complex functional tasks which they used to perform shopping. They may take more time, be less efficient, and make more errors at performing such activities than in the past. Nevertheless, they generally maintain their independence of function in daily life, with minimal aids or assistance.

With respect to dementia, the cognitive changes should be sufficiently mild that there is no evidence of a significant impairment in social or occupational functioning. If an individual has only been evaluated once, change will be inferred from the history and/or evidence that cognitive performance is impaired beyond what would have been expected for that individual.

Cognitive testing is optimal for objectively assessing the degree of cognitive impairment for an individual. Scores on cognitive tests for individuals with MCI are typically 1 to 1.5 standard deviations below the mean for their age and education matched peers on culturally appropriate normative data *(e.g.,* for the impaired domain(s), when available).

Episodic memory (*i.e*., the ability to learn and retain new information) is most commonly seen in MCI patients who subsequently progress to a diagnosis of AD dementia. There are a variety of episodic memory tests that are useful for identifying those MCI patients who have a high likelihood of progressing to AD dementia within a few years. These tests typically assess both immediate and delayed recall, so that it is possible to determine retention over a delay. Many, although not all, of the tests that have proven useful in this regard are wordlist learning tests with multiple trials. Such tests reveal the rate of learning over time, as well as the maximum amount acquired over the course of the learning trials. They are also useful for demonstrating that the individual is, in fact, paying attention to the task on immediate recall, which then can be used as a baseline to assess the relative amount of material retained on delayed recall. Examples of such tests include (but are not limited to: the Free and Cued Selective Reminding Test, the Rey Auditory Verbal Learning Test, and the California Verbal Learning Test. Other episodic memory measures include, but are not limited to: immediate and delayed recall of a paragraph such as the Logical Memory I and II of the Wechsler Memory Scale Revised (or other versions) and immediate and delayed recall of nonverbal materials, such as the Visual Reproduction subtests of the Wechsler Memory Scale-Revised I and II.

Because other cognitive domains can be impaired among individuals with MCI, it is desirable to examine domains in addition to memory. These include, but are not limited to executive functions (*e.g*., set-shifting, reasoning, problem-solving, planning), language (*e.g*., naming, fluency, expressive speech, and comprehension), visuospatial skills, and attentional control (*e.g*., simple and divided attention). Many clinical neuropsychological measures are available to assess these cognitive domains, including (but not limited to the Trail Making Test (executive function), the Boston Naming Test, letter and category fluency (language), figure copying (spatial skills), and digit span forward (attention).

As indicated above, genetic factors can be incorporated into the diagnosis of MCI. If an autosomal dominant form of AD is known to be present (*e.g*., mutation in APP, PS1, PS2), then the development of MCI is most likely the precursor to AD dementia. The large majority of these cases develop early onset AD (*e.g.,* onset below 65 years of age).

In addition, there are genetic influences on the development of late onset AD dementia. For example, the presence of one or two ε4 alleles in the apolipoprotein E (APOE) gene is a genetic variant broadly accepted as increasing risk for late-onset AD dementia. Evidence suggests that an individual who meets the clinical, cognitive, and etiologic criteria for MCI, and is also APOE ε4 positive, is more likely to progress to AD dementia within a few years than an individual without this genetic characteristic. It is believed that additional genes play an important, but smaller role than APOE and also confer changes in risk for progression to AD dementia (*see, e.g.,* Bertram et al., (2010) Neuron, 21: 270-281).

In certain embodiments, subjects suitable for the prophylactic methods described herein (*e.g.*, administration of a C41 compound or formulation, and/or an enantiomer, a mixture of enantiomers, or a mixture of two or more diastereomers thereof; or a pharmaceutically acceptable salt, ester, amide, solvate or hydrate thereof or derivatives thereof) include, but need not be limited to subjects identified having one or more of the core clinical criteria described above and/or subjects identified with one or more "research criteria" for MCI, *e.g.*, as described below.

"Research criteria" for the identification/prognosis of MCI include, but are not limited to biomarkers that increase the likelihood that MCI syndrome is due to the pathophysiological processes of AD. Without being bound to a particular theory, it is believed that the conjoint application of clinical criteria and biomarkers can result in various levels of certainty that the MCI syndrome is due to AD pathophysiological processes. In certain embodiments, two categories of biomarkers have been the most studied and applied to clinical outcomes are contemplated. These include "Aβ" (which includes CSF Aβ₄₂ and/or PET amyloid imaging) and "biomarkers of neuronal injury" (which include, but are not limited to CSF tau/p-tau, hippocampal, or medial temporal lobe atrophy on MRI, and temporoparietal/ precuneus hypometabolism or hypoperfusion on PET or SPECT).

Without being bound to a particular theory, it is believed that evidence of both Aβ, and neuronal injury (either an increase in tau/p-tau or imaging biomarkers in a topographical pattern characteristic of AD), together confers the highest probability that the AD pathophysiological process is present. Conversely, if these biomarkers are negative, this may provide information concerning the likelihood of an alternate diagnosis. It is recognized that biomarker findings may be contradictory and accordingly any biomarker combination is indicative (an indicator) used on the context of a differential diagnosis and not itself dispositive. It is recognized that varying severities of an abnormality may confer different likelihoods or prognoses, that are difficult to quantify accurately for broad application.

For those potential MCI subjects whose clinical and cognitive MCI syndrome is consistent with AD as the etiology, the addition of biomarker analysis effects levels of certainty in the diagnosis. In the most typical example in which the clinical and cognitive syndrome of MCI has been established, including evidence of an episodic memory disorder and a presumed degenerative etiology, the most likely cause is the neurodegenerative process of AD. However, the eventual outcome still has variable degrees of certainty. The likelihood of progression to AD dementia will vary with the severity of the cognitive decline and the nature of the evidence suggesting that AD pathophysiology is the underlying cause. Without being bound to a particular theory it is believed that positive biomarkers reflecting neuronal injury increase the likelihood that progression to dementia will occur within a few years and that positive findings reflecting both Ab accumulation and neuronal injury together confer the highest likelihood that the diagnosis is MCI due to AD.

A positive Aβ biomarker and a positive biomarker of neuronal injury provide an indication that the MCI syndrome is due to AD processes and the subject is well suited for the methods described herein (*e.g*., treatment with a C41 compound).

A positive Aβ biomarker in a situation in which neuronal injury biomarkers have not been or cannot be tested or a positive biomarker of neuronal injury in a situation in which Aβ biomarkers have not been or cannot be tested indicate an intermediate likelihood that the MCI syndrome is due to AD. Such subjects are believed to be well suited for the methods described herein

Negative biomarkers for both Aβ and neuronal injury suggest that the MCI syndrome is not due to AD. In such instances the subjects may not be well suited for the methods described herein.

There is evidence that magnetic resonance imaging can observe deterioration, including progressive loss of gray matter in the brain, from mild cognitive impairment to full-blown Alzheimer disease (*see, e.g*., Whitwell et al., (2008) Neurology 70(7): 512-520). A technique known as PiB PET imaging is used to clearly show the sites and shapes of beta amyloid deposits in living subjects using a C11 tracer that binds selectively to such deposits (*see, e.g.*, Jack et al., (2008) Brain 131(Pt 3): 665-680).

In certain embodiments, MCI is typically diagnosed when there is 1) Evidence of memory impairment; 2) Preservation of general cognitive and functional abilities; and 3) Absence of diagnosed dementia.

In certain embodiments, MCI and stages of Alzheimer's disease can be identified/categorized, in part by Clinical Dementia Rating (CDR) scores. The CDR is a five point scale used to characterize six domains of cognitive and functional performance applicable to Alzheimer disease and related dementias: Memory, Orientation, Judgment & Problem Solving, Community Affairs, Home & Hobbies, and Personal Care. The information to make each rating is obtained through a semi-structured interview of the patient and a reliable informant or collateral source (*e.g*., family member).

The CDR table provides descriptive anchors that guide the clinician in making appropriate ratings based on interview data and clinical judgment. In addition to ratings for each domain, an overall CDR score may be calculated through the use of an algorithm. This score is useful for characterizing and tracking a patient's level of impairment/dementia: 0 = Normal; 0.5 = Very Mild Dementia; 1 = Mild Dementia; 2 = Moderate Dementia; and 3 = Severe Dementia. An illustrative CDR table is shown in Table 2.

**Table 2. Illustrative clinical dementia rating (CDR) table.**

| **Impairment: CDR:** | **None 0** | **Questionable 0.5** | **Mild 1** | **Moderate 2** | **Severe 3** |
|---|---|---|---|---|---|
| Memory | No memory loss or slight inconsistent forgetfulness | Consistent slight forgetfulness; partial recollection of events' "benign" forgetfulness | Moderate memory loss; more marked for recent events; defect interferes with everyday activities | Severe memory loss; only highly learned material retained; new material rapidly lost | Severe memory loss; only fragments remain |
| Orientation | Fully oriented | Fully oriented except for slight difficulty with time relationships | Moderate difficulty with time relationships; oriented for place at examination; may have geographic disorientation elsewhere | Severe difficulty with time relationships; usually disoriented to time, often to place. | Oriented to person only |
| Judgment & Problem Solving | Solves everyday problems & handles business & financial affairs well; judgment good in relation to past performance | Slight impairment in solving problems, similarities, and differences | Moderate difficulty in handling problems, similarities and differences; social judgment usually maintained | Severely impaired in handling problems, similarities and differences; social judgment usually impaired | Unable to make judgments or solve problems |
| Community Affairs | Independent function at usual level in job, shopping, volunteer, and social groups | Slight impairment in these activities | Unable to function independently at these activities although may still be engaged in some; appears normal to casual inspection | No pretense of independent function outside of home | |
| | | | | Appears well enough to be taken to functions outside a family home | Appears too ill to be taken to functions outside a family home. |
| Home and Hobbies | Life at home, hobbies, and intellectual interests well maintained | Life at home, hobbies, and intellectual interests slightly impaired | Mild bit definite impairment of function at home; more difficult chores abandoned; more complicated hobbies and interests abandoned | Only simple chores preserved; very restricted interests, poorly maintained | No significant function in home |
| Personal Care | Fully capable of self-care | | Needs prompting | Requires assistance in dressing, hygiene, keeping of personal effects | Requires much help with personal care; frequent incontinence |

A CDR rating of ~0.5 or ~0.5 to 1.0 is often considered clinically relevant MCI. Higher CDR ratings can be indicative of progression into Alzheimer's disease.

In certain embodiments, administration of one or more agents described herein (*e.g.,* a C41 compound or formulation, and/or an enantiomer, a mixture of enantiomers, or a mixture of two or more diastereomers thereof; or a pharmaceutically acceptable salt, ester, amide, solvate or hydrate thereof or derivatives thereof) is deemed effective when there is a reduction in the CSF of levels of one or more components selected from the group consisting of Tau, phospho-Tau (pTau), APPneo, soluble Aβ40, soluble Aβ42, and/or Aβ42/Aβ40 ratio, and/or when there is a reduction of the plaque load in the brain of the subject, and/or when there is a reduction in the rate of plaque formation in the brain of the subject, and/or when there is an improvement in the cognitive abilities of the subject, and/or when there is a perceived improvement in quality of life by the subject, and/or when there is a significant reduction in clinical dementia rating (CDR), and/or when the rate of increase in clinical dementia rating is slowed or stopped and/or when the progression from MCI to early stage AD is slowed or stopped.

In some embodiments, a diagnosis of MCI can be determined by considering the results of several clinical tests. For example, Grundman, et al., (2004) Arch Neurol 61: 59-66, report that a diagnosis of MCI can be established with clinical efficiency using a simple memory test (paragraph recall) to establish an objective memory deficit, a measure of general cognition (Mini-Mental State Exam (MMSE), discussed in greater detail below) to exclude a broader cognitive decline beyond memory, and a structured clinical interview (CDR) with patients and caregivers to verify the patient's memory complaint and memory loss and to ensure that the patient was not demented. Patients with MCI perform, on average, less than 1 standard deviation (SD) below normal on nonmemory cognitive measures included in the battery. Tests of learning, attention, perceptual speed, category fluency, and executive function may be impaired in patients with MCI, but these are far less prominent than the memory deficit.

### Alzheimer's Disease (AD).

In certain embodiments, the active agent(s) (*e.g.,* a C41 compound or formulation, and/or an enantiomer, a mixture of enantiomers, or a mixture of two or more diastereomers thereof, or a pharmaceutically acceptable salt, ester, amide, solvate or hydrate thereof or derivatives thereof) and/or formulations thereof are contemplated for the treatment of Alzheimer's disease. In such instances the methods described herein are useful in preventing or slowing the onset of Alzheimer's disease (AD), in reducing the severity of AD when the subject has transitioned to clinical AD diagnosis, and/or in mitigating one or more symptoms of Alzheimer's disease.

In particular, where the Alzheimer's disease is early stage, the methods can reduce or eliminate one or more symptoms characteristic of AD and/or delay or prevent the progression from MCI to early or later stage Alzheimer's disease, and/or prevent or delay the progression from an early stage of Alzheimer's disease to a later stage of Alzheimer's disease.

Individuals presently suffering from Alzheimer's disease can be recognized from characteristic dementia, as well as the presence of risk factors described above. In addition, a number of diagnostic tests are available for identifying individuals who have AD. Individuals presently suffering from Alzheimer's disease can be recognized from characteristic dementia, as well as the presence of risk factors described above. In addition, a number of diagnostic tests are available for identifying individuals who have AD. These include measurement of CSF Tau, phospho-tau (pTau), sAPPα, sAPPβ, Aβ40, Aβ42 levels and/or C terminally cleaved APP fragment (APPneo). Elevated Tau, pTau, sAPPβ and/or APPneo, and/or decreased sAPPα, soluble Aβ40 and/or soluble Aβ42 levels, particularly in the context of a differential diagnosis, can signify the presence of AD.

In certain embodiments, subjects amenable to treatment may have Alzheimer's disease. Individuals suffering from Alzheimer's disease can also be diagnosed by Alzheimer's disease and Related Disorders Association (ADRDA) criteria. The NINCDS-ADRDA Alzheimer's criteria were proposed in 1984 by the National Institute of Neurological and Communicative Disorders and Stroke and the Alzheimer's Disease and Related Disorders Association (now known as the Alzheimer's Association) and are among the most used in the diagnosis of Alzheimer's disease (AD). McKhann, et al., (1984) Neurology 34(7): 939-944. According to these criteria, the presence of cognitive impairment and a suspected dementia syndrome should be confirmed by neuropsychological testing for a clinical diagnosis of possible or probable AD. However, histopathologic confirmation (microscopic examination of brain tissue) is generally used for a dispositive diagnosis. The NINCDS-ADRDA Alzheimer's Criteria specify eight cognitive domains that may be impaired in AD: memory, language, perceptual skills, attention, constructive abilities, orientation, problem solving and functional abilities). These criteria have shown good reliability and validity.

Baseline evaluations of patient function can made using classic psychometric measures, such as the Mini-Mental State Exam (MMSE) (Folstein et al., (1975) J. Psychiatric Research 12 (3): 189-198), and the Alzheimer's Disease Assessment Scale (ADAS), which is a comprehensive scale for evaluating patients with Alzheimer's Disease status and function (*see, e.g*., Rosen, et al., (1984) Am. J. Psychiatr., 141: 1356-1364). These psychometric scales provide a measure of progression of the Alzheimer's condition. Suitable qualitative life scales can also be used to monitor treatment. The extent of disease progression can be determined using a Mini-Mental State Exam (MMSE) (see, *e.g*., Folstein, *et al., supra*). Any score greater than or equal to 25 points (out of 30) is effectively normal (intact). Below this, scores can indicate severe (≤9 points), moderate (10-20 points) or mild (21-24 points) Alzheimer's disease.

Alzheimer's disease can be broken down into various stages including: 1) Moderate cognitive decline (mild or early-stage Alzheimer's disease), 2) Moderately severe cognitive decline (moderate or mid-stage Alzheimer's disease), 3) Severe cognitive decline (moderately severe or mid-stage Alzheimer's disease), and 4) Very severe cognitive decline (severe or late-stage Alzheimer's disease) as shown in Table 3.

**Table 3. Illustrative stages of Alzheimer's disease.**

| **Moderate Cognitive Decline (Mild or early stage AD)** | | |
|---|---|---|
| | At this stage, a careful medical interview detects clear-cut deficiencies in the following areas: | |
| | | Decreased knowledge of recent events. |
| | | Impaired ability to perform challenging mental arithmetic. For example, to count backward from 100 by 7s. |
| | | Decreased capacity to perform complex tasks, such as marketing, planning dinner for guests, or paying bills and managing finances. |
| | | Reduced memory of personal history. |
| | | The affected individual may seem subdued and withdrawn, especially in socially or mentally challenging situations. |

| **Moderately severe cognitive decline (Moderate or mid-stage Alzheimer's disease)** | | |
|---|---|---|
| | Major gaps in memory and deficits in cognitive function emerge. Some assistance with day-to-day activities becomes essential. At this stage, individuals may: | |
| | | Be unable during a medical interview to recall such important details as their current address, their telephone number, or the name of the college or high school from which they graduated. |
| | | Become confused about where they are or about the date, day of the week or season. |
| | | Have trouble with less challenging mental arithmetic; for example, |
| | | counting backward from 40 by 4s or from 20 by 2s. |
| | | Need help choosing proper clothing for the season or the occasion. |
| | | Usually retain substantial knowledge about themselves and know their own name and the names of their spouse or children. |
| | | Usually require no assistance with eating or using the toilet. |

| **Severe cognitive decline (Moderately severe or mid-stage Alzheimer's disease)** | | |
|---|---|---|
| | Memory difficulties continue to worsen, significant personality changes may emerge, and affected individuals need extensive help with daily activities. At this stage, individuals may: | |
| | | Lose most awareness of recent experiences and events as well as of their surroundings. |
| | | Recollect their personal history imperfectly, although they generally recall their own name. |
| | | Occasionally forget the name of their spouse or primary caregiver but generally can distinguish familiar from unfamiliar faces. |
| | | such Need help getting dressed properly; without supervision, may make errors as putting pajamas over daytime clothes or shoes on wrong feet. |
| | | Experience disruption of their normal sleep/waking cycle. |
| | | Need help with handling details of toileting (flushing toilet, wiping and disposing of tissue properly). |
| | | Have increasing episodes of urinary or fecal incontinence. |
| | | Experience significant personality changes and behavioral symptoms, including suspiciousness and delusions (for example, believing that their caregiver is an impostor); hallucinations (seeing or hearing things that are not really there); or compulsive, repetitive behaviors such as hand-wringing or tissue shredding. |
| | | Tend to wander and become lost. |

| **Very severe cognitive decline (Severe or late-stage Alzheimer's disease)** | | |
|---|---|---|
| | This is the final stage of the disease when individuals lose the ability to respond to their environment, the ability to speak, and, ultimately, the ability to control movement. | |
| | | Frequently individuals lose their capacity for recognizable speech, although words or phrases may occasionally be uttered. |
| | | Individuals need help with eating and toileting and there is general incontinence. |
| | | Individuals lose the ability to walk without assistance, then the ability to sit without support, the ability to smile, and the ability to hold their head up. |
| | | Reflexes become abnormal and muscles grow rigid. Swallowing is impaired. |

In various embodiments, administration of one or more agents described herein to subjects diagnosed with Alzheimer's disease is deemed effective when the there is a reduction in the CSF of levels of one or more components selected from the group consisting of Tau, phospho-Tau (pTau), APPneo, soluble Aβ40, soluble Aβ42, and/or and Aβ42/Aβ40 ratio, and/or when there is a reduction of the plaque load in the brain of the subject, and/or when there is a reduction in the rate of plaque formation in the brain of the subject, and/or when there is an improvement in the cognitive abilities of the subject, and/or when there is a perceived improvement in quality of life by the subject, and/or when there is a significant reduction in clinical dementia rating (CDR) of the subject, and/or when the rate of increase in clinical dementia rating is slowed or stopped and/or when the progression of AD is slowed or stopped (*e.g.*, when the transition from one stage to another as listed in Table 3 is slowed or stopped).

In certain embodiments, subjects amenable to the present methods generally are free of a neurological disease or disorder other than Alzheimer's disease. For example, in certain embodiments, the subject does not have and is not at risk of developing a neurological disease or disorder such as Huntington's Disease, and/or Parkinson's disease, and/or schizophrenia, and/or psychosis.

In various embodiments, the effectiveness of treatment can be determined by comparing a baseline measure of a parameter of disease before administration of the active agent(s) (*e.g.,* a C41 compound or formulation, and/or an enantiomer, a mixture of enantiomers, or a mixture of two or more diastereomers thereof; or a pharmaceutically acceptable salt, ester, amide, solvate or hydrate thereof or derivatives thereof) is commenced to the same parameter one or more time points after the compound/formulation has been administered. One illustrative, but non-limiting, parameter that can be measured is a biomarker (*e.g*., a peptide oligomer) of APP processing. Such biomarkers include, but are not limited to increased levels of sAPPα, p3 (Aβ 17-42 or Aβ 17-40), sAPPβ, soluble Aβ40, and/or soluble Aβ42 in the blood, plasma, serum, urine, mucous or cerebrospinal fluid (CSF). Detection of increased levels of sAPPα and/or p3, and decreased levels of sAPPβ and/or APPneo is an indicator that the treatment is effective. Conversely, detection of decreased levels of sAPPα and/or p3, and/or increased levels of sAPPβ, APPneo, Tau or phospho-Tau (pTau) is an indicator that the treatment is not effective.

Another parameter to determine effectiveness of treatment is the level of amyloid plaque deposits in the brain. Amyloid plaques can be determined using any method known in the art, *e.g*., as determined by CT, PET, PIB-PET and/or MRI.

In various embodiments, administration of the active agent(s) described herein can result in a reduction in the rate of plaque formation, and even a retraction or reduction of plaque deposits in the brain. Effectiveness of treatment can also be determined by observing a stabilization and/or improvement of cognitive abilities of the subject. Cognitive abilities can be evaluated using any art-accepted method, including for example, Clinical Dementia Rating (CDR), the mini-mental state examination (MMSE) or Folstein test, evaluative criteria listed in the DSM-IV (Diagnostic and Statistical Manual of Mental Disorders, Fourth Edition) or DSM-V, and the like.

In certain embodiments, the monitoring methods can entail determining a baseline value of a measurable biomarker or parameter (*e.g.*, amyloid plaque load or cognitive abilities) in a subject before administering a dosage of the multi-component formulation and optionally one or more pharmaceuticals, and comparing this biomarker or parameter with a value for the same measurable biomarker or parameter after treatment.

In other methods, a control value (e.g., a mean and standard deviation) of the measurable biomarker or parameter is determined for a control population. In certain embodiments, the individuals in the control population have not received prior treatment and do not have AD, MCI, nor are at risk of developing AD or MCI. In such cases, if the value of the measurable biomarker or clinical parameter approaches the control value, then treatment is considered efficacious. In other embodiments, the individuals in the control population have not received prior treatment and have been diagnosed with AD or MCI. In such cases, if the value of the measurable biomarker or clinical parameter approaches the control value, then treatment is considered inefficacious.

In other methods, a subject who is not presently receiving treatment but has undergone a previous course of treatment is monitored for one or more of the biomarkers or clinical parameters to determine whether a resumption of treatment is required. The measured value of one or more of the biomarkers or clinical parameters in the subject can be compared with a value previously achieved in the subject after a previous course of treatment. Alternatively, the value measured in the subject can be compared with a control value (mean plus standard deviation/ ANOVA) determined in population of subjects after undergoing a course of treatment. Alternatively, the measured value in the subject can be compared with a control value in populations of prophylactically treated subjects who remain free of symptoms of disease, or populations of therapeutically treated subjects who show amelioration of disease characteristics. In such cases, if the value of the measurable biomarker or clinical parameter approaches the control value, then treatment is considered efficacious and need not be resumed. In all of these cases, a significant difference relative to the control level (*e.g*., more than a standard deviation) is an indicator that treatment should be resumed in the subject.

In various embodiments, the tissue sample for analysis is typically blood, plasma, serum, urine, mucous or cerebrospinal fluid from the subject.

### Compound C41 and pharmaceutical formulations.

### Compound C41.

In various embodiments, methods are contemplated that use compound C41, or an enantiomer, a mixture of enantiomers, or a mixture of two or more diastereomers thereof, or a pharmaceutically acceptable salt, ester, amide, solvate or hydrate thereof, or derivatives thereof. Thus, in certain embodiments, a compound according to Formula I 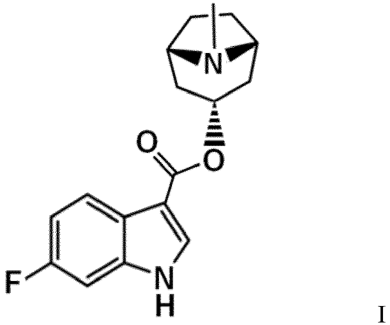 or an enantiomer, a mixture of enantiomers, or a mixture of two or more diastereomers thereof; or a pharmaceutically acceptable salt, ester, amide, solvate or hydrate thereof is provided. In certain embodiments the compound comprises a pharmaceutically acceptable salt (*e.g.*, a salt of an acid selected from the group consisting of 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid (D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (- L), malonic acid, mandelic acid (DL), methanesulfonic acid , naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid (- L), salicylic acid, sebacic acid, stearic acid, succinic acid, sulfuric acid, tartaric acid (+ L), thiocyanic acid, toluenesulfonic acid (*p*), acid undecylenic acid). In certain embodiments the C41 is provided as C41 HCl. It will be recognized that this is not limiting and in various embodiments any of the above acid salt forms are contemplated.

Example 1 and Figure 2 provide one illustrative method of making compound C41 and C41 HCl. Using the teaching provided herein, numerous C41 enantiomera, mixtures of enantiomers, mixtures of two or more diastereomers thereof, other pharmaceutically acceptable salts, esters, amides, solvates, hydrates, or derivatives thereof will be available to those of skill in the art.

In certain embodiments, the C41 derivatives contemplated herein exclude any of the compounds described in U.S. Patent 5,434,161.

### Pharmaceutical formulations.

In certain embodiments, one or more active agents (*e.g.,* a C41 compound or formulation, and/or an enantiomer, a mixture of enantiomers, or a mixture of two or more diastereomers thereof, a pharmaceutically acceptable salt, ester, amide, solvate or hydrate thereof or derivatives thereof) are administered to a mammal in need thereof, *e.g.,* to a mammal at risk for or suffering from a pathology characterized by abnormal processing of amyloid precursor proteins, a mammal at risk for progression of MCI to Alzheimer's disease, and so forth.

The active agent(s) can be administered in the "native" form or, if desired, in the form of salts, esters, amides, derivatives, and the like, provided the salt, ester, amide or derivative is suitable pharmacologically, *e.g.,* effective in the present method(s). Salts, esters, amides and other derivatives of the active agents can be prepared using standard procedures known to those skilled in the art of synthetic organic chemistry and described, for example, by March (1992) Advanced Organic Chemistry; Reactions, Mechanisms and Structure, 4th Ed. N.Y. Wiley-Interscience.

Methods of formulating such derivatives are known to those of skill in the art. For example, a pharmaceutically acceptable salt can be prepared for any compound described herein having a functionality capable of forming a salt, such as the carboxylic acid or tetrazole functionality of the compounds described herein. A pharmaceutically acceptable salt is any salt which retains the activity of the parent compound and does not impart any deleterious or untoward effect on the subject to which it is administered and in the context in which it is administered.

Methods of formulating pharmaceutically active agents as salts, esters, amide, and the like are well known to those of skill in the art. For example, salts can be prepared from the free base using conventional methodology that typically involves reaction with a suitable acid. Generally, the base form of the drug is dissolved in a polar organic solvent such as methanol or ethanol and the acid is added thereto. The resulting salt either precipitates or can be brought out of solution by addition of a less polar solvent. Suitable acids for preparing acid addition salts include, but are not limited to both organic acids, *e.g.,* acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, *e.g.,* hydrochloric acid, hydrobromic acid, sulfuric acid, nitric acid, phosphoric acid, and the like. An acid addition salt can be reconverted to the free base by treatment with a suitable base. Certain particularly preferred acid addition salts of the active agents herein include halide salts, such as may be prepared using hydrochloric or hydrobromic acids. Conversely, preparation of basic salts of the active agents described herein (*e.g.,* C41) are prepared in a similar manner using a pharmaceutically acceptable base such as sodium hydroxide, potassium hydroxide, ammonium hydroxide, calcium hydroxide, trimethylamine, or the like. In certain embodiments basic salts include alkali metal salts, *e.g.,* the sodium salt, and copper salts.

For the preparation of salt forms of basic drugs, the pKa of the counterion is preferably at least about 2 pH units lower than the pKa of the drug. Similarly, for the preparation of salt forms of acidic drugs, the pKa of the counterion is preferably at least about 2 pH units higher than the pKa of the drug. This permits the counterion to bring the solution's pH to a level lower than the pHₘₐₓ to reach the salt plateau, at which the solubility of salt prevails over the solubility of free acid or base. The generalized rule of difference in pKa units of the ionizable group in the active pharmaceutical ingredient (API) and in the acid or base is meant to make the proton transfer energetically favorable. When the pKa of the API and counterion are not significantly different, a solid complex may form but may rapidly disproportionate (*e.g.,* break down into the individual entities of drug and counterion) in an aqueous environment.

In various embodiments, the counterion is a pharmaceutically acceptable counterion. Suitable anionic salt forms include, but are not limited to acetate, benzoate, benzylate, bitartrate, bromide, carbonate, chloride, citrate, edetate, edisylate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate (embonate), phosphate and diphosphate, salicylate and disalicylate, stearate, succinate, sulfate, tartrate, tosylate, triethiodide, valerate, and the like, while suitable cationic salt forms include, but are not limited to aluminum, benzathine, calcium, ethylene diamine, lysine, magnesium, meglumine, potassium, procaine, sodium, tromethamine, zinc, and the like.

Preparation of esters typically involves functionalization of hydroxyl and/or carboxyl groups that are present within the molecular structure of the active agent. In certain embodiments, the esters are typically acyl-substituted derivatives of free alcohol groups, *e.g.*, moieties that are derived from carboxylic acids of the formula RCOOH where R is alky, and preferably is lower alkyl. Esters can be reconverted to the free acids, if desired, by using conventional hydrogenolysis or hydrolysis procedures.

Amides can also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine.

In various embodiments, the active agents identified herein are useful for parenteral, topical, oral, nasal (or otherwise inhaled), rectal, or local administration, such as by aerosol or transdermally, for prophylactic and/or therapeutic treatment of one or more of the pathologies/indications described herein (*e.g.*, amyloidogenic pathologies).

The active agents described herein (*e.g.*, a C41 compound, and/or an enantiomer, a mixture of enantiomers, or a mixture of two or more diastereomers thereof; or a pharmaceutically acceptable salt, ester, amide, solvate or hydrate thereof or derivatives thereof) can also be combined with a pharmaceutically acceptable carrier (excipient) to form a pharmacological composition. Pharmaceutically acceptable carriers can contain one or more physiologically acceptable compound(s) that act, for example, to stabilize the composition or to increase or decrease the absorption of the active agent(s). Physiologically acceptable compounds can include, for example, carbohydrates, such as glucose, sucrose, or dextrans, antioxidants, such as ascorbic acid or glutathione, chelating agents, low molecular weight proteins, protection and uptake enhancers such as lipids, compositions that reduce the clearance or hydrolysis of the active agents, or excipients or other stabilizers and/or buffers.

Other physiologically acceptable compounds, particularly of use in the preparation of tablets, capsules, gel caps, and the like include, but are not limited to binders, diluent/fillers, disentegrants, lubricants, suspending agents, and the like.

In certain embodiments, to manufacture an oral dosage form (*e.g*., a tablet), an excipient (*e.g.*, lactose, sucrose, starch, mannitol, *etc*.), an optional disintegrator (*e.g.,* calcium carbonate, carboxymethylcellulose calcium, sodium starch glycollate, crospovidone *etc*.), a binder (*e.g.,* alpha-starch, gum arabic, microcrystalline cellulose, carboxymethylcellulose, polyvinylpyrrolidone, hydroxypropylcellulose, cyclodextrin, *etc*.), and an optional lubricant (*e.g*., talc, magnesium stearate, polyethylene glycol 6000, *etc*.), for instance, are added to the active component or components (*e.g*., a C41 compound or formulation, and/or an enantiomer, a mixture of enantiomers, or a mixture of two or more diastereomers thereof, a pharmaceutically acceptable salt, ester, amide, solvate, hydrate thereof or derivatives thereof) and the resulting composition is compressed. Where necessary the compressed product is coated, *e.g*., known methods for masking the taste or for enteric dissolution or sustained release. Suitable coating materials include, but are not limited to ethyl-cellulose, hydroxymethylcellulose, polyoxyethylene glycol, cellulose acetate phthalate, hydroxypropylmethylcellulose phthalate, and Eudragit (Rohm & Haas, Germany; methacrylic-acrylic copolymer).

Other physiologically acceptable compounds include wetting agents, emulsifying agents, dispersing agents or preservatives that are particularly useful for preventing the growth or action of microorganisms. Various preservatives are well known and include, for example, phenol and ascorbic acid. One skilled in the art would appreciate that the choice of pharmaceutically acceptable carrier(s), including a physiologically acceptable compound depends, for example, on the route of administration of the active agent(s) and on the particular physio-chemical characteristics of the active agent(s).

In certain embodiments, the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

The pharmaceutical compositions can be administered in a variety of unit dosage forms depending upon the method of administration. Suitable unit dosage forms, include, but are not limited to powders, tablets, pills, capsules, lozenges, suppositories, patches, nasal sprays, injectable, implantable sustained-release formulations, mucoadherent films, topical varnishes, lipid complexes, *etc.*

Pharmaceutical compositions comprising the active agents (*e.g.*, a C41 compound or formulation, and/or an enantiomer, a mixture of enantiomers, or a mixture of two or more diastereomers thereof, a pharmaceutically acceptable salt, ester, amide, solvate, hydrate thereof or derivatives thereof) described herein can be manufactured by means of conventional mixing, dissolving, granulating, dragee-making, levigating, emulsifying, encapsulating, entrapping or lyophilizing processes. Pharmaceutical compositions can be formulated in a conventional manner using one or more physiologically acceptable carriers, diluents, excipients or auxiliaries that facilitate processing of the active agents into preparations that can be used pharmaceutically. Proper formulation is dependent upon the route of administration chosen.

For topical administration the active agents described herein can be formulated as solutions, gels, ointments, creams, suspensions, and the like as are well-known in the art. Systemic formulations include, but are not limited to, those designed for administration by injection, *e.g.,* subcutaneous, intravenous, intramuscular, intrathecal or intraperitoneal injection, as well as those designed for transdermal, transmucosal oral or pulmonary administration. For injection, the active agents described herein can be formulated in aqueous solutions, preferably in physiologically compatible buffers such as Hanks solution, Ringer's solution, or physiological saline buffer and/or in certain emulsion formulations. The solution can contain formulatory agents such as suspending, stabilizing and/or dispersing agents. In certain embodiments, the active agent(s) can be provided in powder form for constitution with a suitable vehicle, *e.g.*, sterile pyrogen-free water, before use. For transmucosal administration, penetrants appropriate to the barrier to be permeated can be used in the formulation. Such penetrants are generally known in the art.

For oral administration, the compounds can be readily formulated by combining the active agent(s) with pharmaceutically acceptable carriers well known in the art. Such carriers enable the compounds described herein (*e.g*., a C41 compound or formulation, and/or an enantiomer, a mixture of enantiomers, or a mixture of two or more diastereomers thereof, a pharmaceutically acceptable salt, ester, amide, solvate, hydrate thereof or derivatives thereof) be formulated as tablets, pills, dragees, capsules, liquids, gels, syrups, slurries, suspensions and the like, for oral ingestion by a patient to be treated. For oral solid formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; and binding agents. If desired, disintegrating agents may be added, such as the cross-linked polyvinylpyrrolidone, agar, or alginic acid or a salt thereof such as sodium alginate. If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, *etc.* Additionally, flavoring agents, preservatives, coloring agents and the like can be added. For buccal administration, the compositions may take the form of tablets, lozenges, *etc.* formulated in conventional manner.

For administration by inhalation, the active agent(s) are conveniently delivered in the form of an aerosol spray from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.,* gelatin for use in an inhaler or insufflator may be formulated containing a powder mix of the compound and a suitable powder base such as lactose or starch.

In various embodiments, the active agent(s) can be formulated in rectal or vaginal compositions such as suppositories or retention enemas, *e.g.,* containing conventional suppository bases such as cocoa butter or other glycerides.

In addition to the formulations described previously, the compounds may also be formulated as a depot preparation. Such long acting formulations can be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Alternatively, other pharmaceutical delivery systems can be employed. Liposomes and emulsions are well known examples of delivery vehicles that may be used to protect and deliver pharmaceutically active compounds. Certain organic solvents such as dimethylsulfoxide also can be employed, although usually at the cost of greater toxicity. Additionally, the compounds may be delivered using a sustained-release system, such as semipermeable matrices of solid polymers containing the therapeutic agent. Various uses of sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

In certain embodiments, the active agents described herein are administered orally. This is readily accomplished by the use of tablets, caplets, lozenges, liquids, and the like.

In certain embodiments, the active agents described herein are administered systemically (*e.g*., orally, or as an injectable) in accordance with standard methods well known to those of skill in the art. In other embodiments, the agents can also be delivered through the skin using conventional transdermal drug delivery systems, *e.g.,* transdermal "patches" wherein the active agent(s) are typically contained within a laminated structure that serves as a drug delivery device to be affixed to the skin. In such a structure, the drug composition is typically contained in a layer, or "reservoir," underlying an upper backing layer. It will be appreciated that the term "reservoir" in this context refers to a quantity of "active ingredient(s)" that is ultimately available for delivery to the surface of the skin. Thus, for example, the "reservoir" may include the active ingredient(s) in an adhesive on a backing layer of the patch, or in any of a variety of different matrix formulations known to those of skill in the art. The patch may contain a single reservoir, or it may contain multiple reservoirs.

In one illustrative embodiment, the reservoir comprises a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are present as separate and distinct layers, with the adhesive underlying the reservoir which, in this case, may be either a polymeric matrix as described above, or it may be a liquid or hydrogel reservoir, or may take some other form. The backing layer in these laminates, which serves as the upper surface of the device, preferably functions as a primary structural element of the "patch" and provides the device with much of its flexibility. The material selected for the backing layer is preferably substantially impermeable to the active agent(s) and any other materials that are present.

In certain embodiments, one or more active agents described herein can be provided as a "concentrate", *e.g.,* in a storage container (*e.g.*, in a premeasured volume) ready for dilution, or in a soluble capsule ready for addition to a volume of water, alcohol, hydrogen peroxide, or other diluent.

In certain embodiments, the active agents described herein (*e.g.*, one or more esters described above) are preferably suitable for oral administration. In various embodiments, the active agent(s) in the oral compositions can be either coated or noncoated. The preparation of enteric-coated particles is disclosed for example in U.S. Pat. Nos. 4,786,505 and 4,853,230.

In various embodiments, compositions contemplated herein typically comprise one or more of the various active agents (*e.g*., a C41 compound or formulation, and/or an enantiomer, a mixture of enantiomers, or a mixture of two or more diastereomers thereof, a pharmaceutically acceptable salt, ester, amide, solvate, hydrate thereof or derivatives thereof) described herein in an effective amount to achieve a pharmacological effect or therapeutic improvement without undue adverse side effects. Various effects deemed therapeutic are described above. Illustrative pharmacological effects or therapeutic improvements include, but are not limited to a reduction in the CSF of levels of one or more components selected from the group consisting of Tau, phospho-Tau (pTau), APPneo, soluble Aβ40 and soluble Aβ42, and/or when a reduction of the plaque load in the brain of the subject, and/or a reduction in the rate of plaque formation in the brain of the subject, and/or an improvement in the cognitive abilities of the subject, and/or a perceived improvement in quality of life by the subject, and/or a significant reduction in clinical dementia rating (CDR) of the subject, and/or a slowing in the rate of increase in clinical dementia rating, and/or when a slowing or stopping in the progression of AD (*e.g.*, when the transition from one stage to another as listed in Table 3 is slowed or stopped).

In various embodiments, the typical daily dose of compound(s) varies and will depend on various factors such as the individual requirements of the patients and the disease to be treated. In general, the daily dose of compounds can be in the range of 1-1,000 mg or 1-800 mg, or 1-600 mg, or 1-500 mg, or 1-400 mg. In one illustrative standard approximate amount of the C41 compounds described above present in the composition can be typically about 1 to 1,000 mg, more preferably about 5 to 500 mg, and most preferably about 10 to 100 mg administered once a day, in certain embodiments, administered twice a day, in certain embodiments, administered 3 times/day, and in certain embodiments, administered 4, or 6, or 6 or 7, or 8 times/day.

The active ingredients of the are preferably formulated in a single oral dosage form containing all active ingredients. Such oral formulations include solid and liquid forms. It is noted that solid formulations typically provide improved stability as compared to liquid formulations and can often afford better patient compliance.

In one illustrative embodiment, the one or more of the various C41 compounds described above are formulated in a single solid dosage form such as single- or multi-layered tablets, suspension tablets, effervescent tablets, powder, pellets, granules or capsules comprising multiple beads as well as a capsule within a capsule or a double chambered capsule. In another embodiment, the active agents may be formulated in a single liquid dosage form such as suspension containing all active ingredients or dry suspension to be reconstituted prior to use.

In certain embodiments, the compound(s) are formulated as enteric-coated delayed-release granules or as granules coated with non-enteric time-dependent release polymers in order to avoid contact with the gastric juice. Non-limiting examples of suitable pH-dependent enteric-coated polymers are: cellulose acetate phthalate, hydroxypropylmethylcellulose phthalate, polyvinylacetate phthalate, methacrylic acid copolymer, shellac, hydroxypropylmethylcellulose succinate, cellulose acetate trimellitate, and mixtures of any of the foregoing. A suitable commercially available enteric material, for example, is sold under the trademark EUDRAGIT L 100-55^{®}. This coating can be spray coated onto a substrate.

Illustrative non-enteric-coated time-dependent release polymers include, for example, one or more polymers that swell in the stomach via the absorption of water from the gastric fluid, thereby increasing the size of the particles to create thick coating layer. The time-dependent release coating generally possesses erosion and/or diffusion properties that are independent of the pH of the external aqueous medium. Thus, the active ingredient is slowly released from the particles by diffusion or following slow erosion of the particles in the stomach.

Illustrative non-enteric time-dependent release coatings are for example: film-forming compounds such as cellulosic derivatives, such as methylcellulose, hydroxypropyl methylcellulose (HPMC), hydroxyethylcellulose, and/or acrylic polymers including the non-enteric forms of the EUDRAGIT^{®} brand polymers. Other film-forming materials can be used alone or in combination with each other or with the ones listed above. These other film forming materials generally include, for example, poly(vinylpyrrolidone), Zein, poly(ethylene glycol), poly(ethylene oxide), poly(vinyl alcohol), poly(vinyl acetate), and ethyl cellulose, as well as other pharmaceutically acceptable hydrophilic and hydrophobic film-forming materials. These film-forming materials may be applied to the substrate cores using water as the vehicle or, alternatively, a solvent system. Hydroalcoholic systems may also be employed to serve as a vehicle for film formation.

Other materials suitable for making the time-dependent release coating of the compounds described herein include, by way of example and without limitation, water soluble polysaccharide gums such as carrageenan, fucoidan, gum ghatti, tragacanth, arabinogalactan, pectin, and xanthan; water-soluble salts of polysaccharide gums such as sodium alginate, sodium tragacanthin, and sodium gum ghattate; water-soluble hydroxyalkylcellulose wherein the alkyl member is straight or branched of 1 to 7 carbons such as hydroxymethylcellulose, hydroxyethylcellulose, and hydroxypropylcellulose; synthetic water-soluble cellulose-based lamina formers such as methyl cellulose and its hydroxyalkyl methylcellulose cellulose derivatives such as a member selected from the group consisting of hydroxyethyl methylcellulose, hydroxypropyl methylcellulose, and hydroxybutyl methylcellulose; other cellulose polymers such as sodium carboxymethylcellulose; and other materials known to those of ordinary skill in the art. Other lamina forming materials that can be used for this purpose include, but are not limited to poly(vinylpyrrolidone), polyvinylalcohol, polyethylene oxide, a blend of gelatin and polyvinyl-pyrrolidone, gelatin, glucose, saccharides, povidone, copovidone, poly(vinylpyrrolidone)-poly(vinyl acetate) copolymer.

While the compositions and methods are described herein with respect to use in humans, they are also suitable for animal, *e.g.,* veterinary use. Thus certain illustrative organisms include, but are not limited to humans, non-human primates, canines, equines, felines, porcines, ungulates, largomorphs, and the like.

The foregoing formulations and administration methods are intended to be illustrative and not limiting. It will be appreciated that, using the teaching provided herein, other suitable formulations and modes of administration can be readily devised.

### Combined treatment methods and combined formulations

In certain instances, one or more of the active agents described above (*e.g*., a C41 compound or formulation, and/or an enantiomer, a mixture of enantiomers, or a mixture of two or more diastereomers thereof, a pharmaceutically acceptable salt, ester, amide, solvate, hydrate thereof or derivatives thereof) are administered in conjunction with one or more additional active agent that are known, or believed, to have utility in the treatment of neurodegenerative diseases including, but not limited to Alzheimer's disease, age-related cognitive impairment, MCI, and the like. The two agents (*e.g*., C41 compound and additional agent) can be administered simultaneously or sequentially. When administered sequentially the two agents are typically administered so that both achieve a physiologically relevant concentration and/or effect over a similar time period (*e.g*., so that both agents are active at some common time).

In certain instances, one or more of the active agents described above (*e.g*., a C41 compound or formulation, and/or an enantiomer, a mixture of enantiomers, or a mixture of two or more diastereomers thereof; or a pharmaceutically acceptable salt, ester, amide, solvate or hydrate thereof or derivatives thereof) are administered before the one or more additional active agents or they are administered after the one or more additional active agents. In certain embodiments one or more of the active agents described above (*e.g*., a C41 compound or formulation, and/or an enantiomer, a mixture of enantiomers, or a mixture of two or more diastereomers thereof; or a pharmaceutically acceptable salt, ester, amide, solvate or hydrate thereof or derivatives thereof) are administered simultaneously with one or more additional active agents and in such instances may be formulated as a compound formulation.

Suitable additional active agents include, but are not limited to, Donepezil (*e.g*., Aricept), Rivastigmine (*e.g*., EXELON^{®}), Galantamine (*e.g*., RAZADINE^{®}), Tacrine (*e.g*., COGNEX^{®}), Memantine (*e.g*., NAMENDA^{®}), Solanezumab, Bapineuzmab, Alzemed, Flurizan, ELND005, Valproate, Semagacestat, Rosiglitazone, Phenserine, Cernezumab, Dimebon, EGCg, Gammagard, PBT2, PF04360365, NIC5-15, Bryostatin-1, AL-108, Nicotinamide, EHT-0202, BMS708163, NP12, Lithium, ACC001, AN1792, ABT089, NGF, CAD106, AZD3480, SB742457, AD02, Huperzine-A, EVP6124, PRX03140, PUPA, HF02, MEM3454, TTP448, PF-04447943, Ent., GSK933776, MABT5102A, Talsaclidine, UB311, Begacestat, R1450, PF3084014, V950, E2609, MK0752, CTS21166, AZD-3839, LY2886721, CHF5074, anti-inflammatories (*e.g.*, Flurizan (Myriad Genetics), Dapsone, anti-TNF antibodies (*e.g.*, etanercept (Amgen/Pfizer)), and the like), statins (*e.g.*, atorvastatin (LIPITOR^{®}), simvastatin (ZOCOR^{®}, *etc*.), BACE inhibitors and the like. In certain embodiments, treatment methods comprising administration of one or more C41 compounds (a C41 compound or formulation, and/or an enantiomer, a mixture of enantiomers, or a mixture of two or more diastereomers thereof, a pharmaceutically acceptable salt, ester, amide, solvate, hydrate thereof or derivatives thereof) in conjunction with any one of the foregoing additional active agents is contemplated.

In certain embodiments, treatment methods comprising administration of one or more C41 compounds (a C41 compound or formulation, and/or an enantiomer, a mixture of enantiomers, or a mixture of two or more diastereomers thereof, a pharmaceutically acceptable salt, ester, amide, solvate, hydrate thereof or derivatives thereof) in conjunction with additional therapeutic agents such as disulfiram and/or analogues thereof, honokiol and/or analogues thereof, tropisetron and/or analogues thereof, nimetazepam and/or analogues thereof (*e.g.*, as described in USSN 13/213,960 (U.S. Patent Publication No: US-2012-0071468-A1), and PCT/US2011/048472 (PCT Publication No: WO 2012/024616)) are contemplated. In certain embodiments the treatment method comprises administration of tropisetron in conjunction with of one or more C41 compounds described herein.

In certain embodiments, combination formulations comprising one or more C41 compounds and the like described herein in combination with additional therapeutic agents such as disulfiram and/or analogues thereof, honokiol and/or analogues thereof, tropisetron and/or analogues thereof, nimetazepam and/or analogues thereof (*e.g.*, as described in USSN 13/213,960 (U.S. Patent Publication No: US-2012-0071468-A1), and PCT/US2011/048472 (PCT Publication No: WO 2012/024616)) are contemplated. In certain embodiments the combination formulation comprises C41 in combination with tropisetron and/or of one or more tropinol esters, related esters, derivatives thereof, analogs thereof, polymorphs thereof (*e.g*. as described in PCT/US2012/049223), and the like is contemplated.

### Assay Systems to Evaluate APP processing

Without being bound to a particular theory, it is believed that, in certain embodiments, the C41 compounds and formulations described herein promote processing of APP by the nonamyloidogenic pathway and/or reduce or inhibit processing of APP by the amyloidogenic pathway. In the nonamyloidogeic pathway, APP is first cleaved by α-secretase within the Aβ sequence, releasing the soluble APPα ectodomain ("sAPPα"). In contrast, the amyloidogenic pathway is initiated when β-secretase cleaves APP at the amino terminus of the Aβ, thereby releasing the soluble APPβ ectodomain ("sAPP(β"). APP processing by the nonamyloidogenic and amyloidogenic pathways is known in the art and reviewed, *e.g.*, by Xu (2009) J. Alzheimer's Dis., 16(2):211-224 and De Strooper et al., (2010) Nat Rev Neurol., 6(2): 99-107.

One method to evaluate the efficacy of the C41 compounds described herein is to determine whether or not the compound(s) in question produce a reduction or elimination in the level of APP processing by the amyloidogenic pathway, *e.g.*, a reduction or elimination in the level of APP processing by β-secretase cleavage. Assays for determining the extent of APP cleavage at the β-secretase cleavage site are well known in the art. Illustrative assays, are described, for example, in U.S. Pat. Nos. 5,744,346 and 5,942,400. Kits for determining the presence and levels in a biological sample of sAPPα and sAPPβ, as well as APPneo and Aβ commercially available, *e.g.*, from PerkinElmer and Enzo Life Sciences.

### Cell Free Assays

Illustrative assays that can be used to evaluate the inhibitory activity of a C41 compound are described, for example, in PCT Publication Nos: WO 2000/017369, and WO 2000/003819, and in U.S. Patent Nos: 5,942,400 and 5,744,346. In certain embodiments, such assays can be performed in cell-free incubations or in cellular incubations using cells expressing an α-secretase and/or β-secretase and an APP substrate having α-secretase and β-secretase cleavage sites.

One illustrative assay, test the compound(s) of interest utilizing an APP substrate containing α-secretase and β-secretase cleavage sites of APP, for example, a complete APP or variant, an APP fragment, or a recombinant or synthetic APP substrate containing the amino acid sequence: KM-DA or NL-DA, which is incubated in the presence of an α-secretase and/or β-secretase enzyme, a fragment thereof, or a synthetic or recombinant polypeptide variant having α-secretase or β-secretase activity and effective to cleave the α-secretase or β-secretase cleavage sites of APP, under incubation conditions suitable for the cleavage activity of the enzyme. Suitable substrates optionally include derivatives that may be fusion proteins or peptides that contain the substrate peptide and a modification useful to facilitate the purification or detection of the peptide or its α-secretase and/or β-secretase cleavage products. Useful modifications include the insertion of a known antigenic epitope for antibody binding; the linking of a label or detectable moiety, the linking of a binding substrate, and the like.

Suitable incubation conditions for a cell-free *in vitro* assay include, for example, approximately 200 nanomolar to 10 micromolar substrate, approximately 10 to 200 picomolar enzyme, and approximately 0.1 nanomolar to 10 micromolar C41 compound in aqueous solution, at an approximate pH of 4-7, at approximately 37°C, for a time period of approximately 10 minutes to 3 hours. These incubation conditions are illustrative only, and can be varied as required for the particular assay components and/or desired measurement system. Optimization of the incubation conditions for the particular assay components can account for the specific α-secretase and/or β-secretase enzyme used and its pH optimum, any additional enzymes and/or markers that might be used in the assay, and the like. Such optimization is routine and does not require undue experimentation.

One useful assay utilizes a fusion peptide having maltose binding protein (MBP) fused to the C-terminal 125 amino acids of APP-SW. The MBP portion is captured on an assay substrate by anti-MBP capture antibody. Incubation of the captured fusion protein in the presence of α-secretase and/or β-secretase results in cleavage of the substrate at the α-secretase and/or β-secretase cleavage sites, respectively. Analysis of the cleavage activity can be, for example, by immunoassay of cleavage products. One such immunoassay detects a unique epitope exposed at the carboxy terminus of the cleaved fusion protein, for example, using the antibody SW192. This assay is described, for example, in U.S. Patent No: 5,942,400.

### Cellular Assays

Numerous cell-based assays can be used to evaluate the effect of the compounds described herein on the ratio of relative α-secretase activity to β-secretase activity and/or on the processing of APP to release amyloidogenic versus nonamyloidogenic Aβ oligomers. Contact of an APP substrate with an α-secretase and/or β-secretase enzyme within the cell and in the presence or absence of compound(s) in question can be used to demonstrate α-secretase and/or β-secretase inhibitory activity of the compound(s). Preferably, the assay in the presence of compound(s) provides at least about 30%, most preferably at least about 50% inhibition of the enzymatic activity, as compared with a non-inhibited control.

In one illustrative embodiment, cells that naturally express α-secretase and/or β-secretase are used. Alternatively, cells can be modified to express a recombinant α-secretase and/or β-secretase or synthetic variant enzymes, as discussed above. In certain embodiments, the APP substrate can be added to the culture medium and in certain embodiments, the substrate is preferably expressed in the cells. Cells that naturally express APP, variant or mutant forms of APP, or cells transformed to express an isoform of APP, mutant or variant APP, recombinant or synthetic APP, APP fragment, or synthetic APP peptide or fusion protein containing the α-secretase and/or β-secretase APP cleavage sites can be used, provided that the expressed APP is permitted to contact the enzyme and enzymatic cleavage activity can be analyzed.

Human cell lines that normally process Aβ from APP provide a useful means to assay inhibitory activities of the compound(s) described herein. Production and release of Aβ and/or other cleavage products into the culture medium can be measured, for example by immunoassay, such as Western blot or enzyme-linked immunoassay (EIA) such as by ELISA.

In certain embodiments, cells expressing an APP substrate and an active α-secretase and/or β-secretase can be incubated in the presence of the compound(s) being tested to demonstrate the effect of the compound(s) on relative enzymatic activity of the α-secretase and/or β-secretase as compared with a control. Relative activity of the α-secretase to the β-secretase can be measured by analysis of one or more cleavage products of the APP substrate. For example, inhibition of β-secretase activity against the substrate APP would be expected to decrease release of specific β-secretase induced APP cleavage products such as Aβ, sAPPβ and APPneo. Promotion or enhancement of α-secretase activity against the substrate APP would be expected to increase release of specific α-secretase induced APP cleavage products such as sAPPα and p3 peptide.

Although both neural and non-neural cells process and release Aβ, levels of endogenous β-secretase activity are low and often difficult to detect by EIA. The use of cell types known to have enhanced beta-secretase activity, enhanced processing of APP to Aβ, and/or enhanced production of Aβ are therefore preferred. For example, transfection of cells with the Swedish Mutant form of APP (APP-SW); with APP-KK (APP containing an ER retention signal (-KKQN-, (SEQ ID NO:1)) appended to the C terminus of APP), or with APP-SW-KK provides cells having enhanced beta-secretase activity and producing amounts of Aβ that can be readily measured.

In such assays, for example, the cells expressing APP, α-secretase and/or β-secretase are incubated in a culture medium under conditions suitable for α-secretase and/or β-secretase enzymatic activity at its cleavage site on the APP substrate. On exposure of the cells to the C41 compound (or formulation, and/or an enantiomer, a mixture of enantiomers, or a mixture of two or more diastereomers thereof; or a pharmaceutically acceptable salt, ester, amide, solvate or hydrate thereof or derivatives thereof), the amount of Aβ released into the medium and/or the amount of CTF99 fragments of APP in the cell lysates is reduced as compared with the control. The cleavage products of APP can be analyzed, for example, by immune reactions with specific antibodies, as discussed above.

In certain embodiments, preferred cells for analysis of α-secretase and/or β-secretase activity include primary human neuronal cells, primary transgenic animal neuronal cells where the transgene is APP, and other cells such as those of a stable 293 cell line expressing APP, for example, APP-SW.

### In vivo Assays: Animal Models

Various animal models can be used to analyze the effect of a C41 compound described herein on the relative alpha-secretase and/or beta-secretase activity and/or processing of APP to release Aβ. For example, transgenic animals expressing APP substrate, α-secretase and/or β-secretase enzyme can be used to demonstrate inhibitory activity of the C41 compound. Certain transgenic animal models have been described, for example, in U.S. Pat. Nos. 5,877,399; 5,612,486; 5,387,742; 5,720,936; 5,850,003; 5,877,015, and 5,811,633, and in Games et al., (1995) Nature 373: 523-527. Preferred are animals that exhibit characteristics associated with the pathophysiology of AD. Administration of the C41 compound to the transgenic mice described herein provides an alternative method for demonstrating the inhibitory activity of the compound(s) in question. In certain embodiments, administration of the C41 compound in a pharmaceutically effective carrier and via an administrative route that reaches the target tissue in an appropriate therapeutic amount is preferred.

Inhibition of β-secretase mediated cleavage of APP at the β-secretase cleavage site and of Aβ release can be analyzed in these animals by measure of cleavage fragments in the animal's body fluids such as cerebral fluid or tissues. Likewise, promotion or enhancement of α-secretase mediated cleavage of APP at the α-secretase cleavage site and of release of sAPPα can be analyzed in these animals by measure of cleavage fragments in the animal's body fluids such as cerebral fluid or tissues. In certain embodiments, analysis of brain tissues for Aβ deposits or plaques is preferred.

In certain illustrative assays, an APP substrate is contacted with an α-secretase and/or β-secretase enzyme in the presence of the C41 compound under conditions sufficient to permit enzymatic mediated cleavage of APP and/or release of Aβ from the substrate. The C41 compound is deemed effective when it reduces β-secretase-mediated cleavage of APP at the β-secretase cleavage site and/or reduces released amounts of Aβ. In certain embodiments the C41 related compounds are also deemed effective if they enhance α-secretase-mediated cleavage of APP at the α-secretase cleavage site and to increase released amounts of sAPPα and/or to reduce Aβ deposition in brain tissues of the animal, and to reduce the number and/or size of beta amyloid (Aβ) plaques.

### Methods of Monitoring Clinical Efficacy

In certain embodiments, clinical efficacy can be monitored using any method known in the art. Measurable biomarkers to monitor efficacy include, but are not limited to, monitoring blood, plasma, serum, mucous or cerebrospinal fluid (CSF) levels of sAPPα, sAPPβ, Aβ42, Aβ40, APPneo and p3 (*e.g*., Aβ17-42 or Aβ17-40). Detection of increased levels of sAPPα and/or p3 and decreased levels of sAPPβ and APPneo are indicators that the treatment or prevention regime is efficacious. Conversely, detection of decreased levels of sAPPα and/or p3 and increased levels of sAPPβ, Aβ42 and APPneo are indicators that the treatment or prevention regime is not efficacious. Other biomarkers include Tau and phospho-Tau (pTau). Detection of decreased levels of Tau and pTau are indicators that the treatment or prevention regime is efficacious.

Efficacy can also be determined by measuring amyloid plaque load in the brain. The treatment or prevention regime is considered efficacious when the amyloid plaque load in the brain does not increase or is reduced. Conversely, the treatment or prevention regime is considered inefficacious when the amyloid plaque load in the brain increases. Amyloid plaque load can be determined using any method known in the art, *e.g.*, including magnetic resonance imaging (MRI).

Efficacy can also be determined by measuring the cognitive abilities of the subject. Cognitive abilities can be measured using any method known in the art. One test is the clinical dementia rating (CDR) described above, while another is the mini mental state examination (MMSE) (Folstein, et al., J. Psychiatric Res. 12 (3): 189-198). In certain embodiments, subjects who maintain the same score or who achieve a higher score on a CDR and/or on an MMSE indicate that the treatment or prevention regime is efficacious. Conversely, subjects who score lower on a CDR and/or on an MMSE indicate that the treatment or prevention regime has not been efficacious.

In certain embodiments, the monitoring methods can entail determining a baseline value of a measurable biomarker or parameter (*e.g*., amyloid plaque load or cognitive ability) in a subject before administering a dosage of C41 compound (*e.g.*, a C41 compound or formulation, and/or an enantiomer, a mixture of enantiomers, or a mixture of two or more diastereomers thereof; or a pharmaceutically acceptable salt, ester, amide, solvate or hydrate thereof or derivatives thereof) and comparing this with a value for the same measurable biomarker or parameter after treatment.

In other methods, a control value (*e.g.*, a mean and standard deviation) of the measurable biomarker or parameter is determined for a control population. In certain embodiments, the individuals in the control population have not received prior treatment and do not have AD, MCI, nor are at risk of developing AD or MCI. In such cases, if the value of the measurable biomarker or clinical parameter approaches the control value, then treatment is considered efficacious. In other embodiments, the individuals in the control population have not received prior treatment and have been diagnosed with AD or MCI. In such cases, if the value of the measurable biomarker or clinical parameter approaches the control value, then treatment is considered inefficacious.

In other methods, a subject who is not presently receiving treatment, but has undergone a previous course of treatment is monitored for one or more of the biomarkers or clinical parameters to determine whether a resumption of treatment is required. The measured value of one or more of the biomarkers or clinical parameters in the subject can be compared with a value previously achieved in the subject after a previous course of treatment. Alternatively, the value measured in the subject can be compared with a control value (mean plus standard deviation) determined in population of subjects after undergoing a course of treatment. Alternatively, the measured value in the subject can be compared with a control value in populations of prophylactically treated subjects who remain free of symptoms of disease, or populations of therapeutically treated subjects who show amelioration of disease characteristics. In such cases, if the value of the measurable biomarker or clinical parameter approaches the control value, then treatment is considered efficacious and a decision not to resume treatment can be considered/evaluated. In all of these cases, a significant difference relative to the control level (*e.g.*, more than a standard deviation) is an indicator that resumption of the subject should be considered.

In certain embodiments, the tissue sample for analysis is typically blood, plasma, serum, mucous or cerebrospinal fluid from the subject.

### EXAMPLES

The following examples are offered to illustrate, but not to limit the claimed invention.

### Example 1

### Synthesis of C41 hydrochloride salt

In certain embodiments the HCl salt of C41 can be synthesized according to Scheme 1 shown in Figure 2. Reagents and reaction conditions were: (a) i. DMF, 0C,3h ii. 4M NaOH, reflux, 4h ; (b) oxalyl chloride, DCM, tropine, overnight; (c) 4MHCl, 1,4-dioxan, 2h.

### 6-fluoroiindole-3-carboxylic acid (2):

Trifluoroacetic anhydride (9.009 ml, 64.8 mmol, 2.2 equiv.) was added drop wise to a stirring solution of 6-fluoroindole (4.000 g, 29.6 mmol, 1.0 equiv.) in DMF (25 mL) at 0°C (external ice bath temperature). After 3 hours in the ice bath the mixture was quenched with 40 mL of 2N sodium carbonate. The precipitate was collected by filtration and washed with water. The solid was taken up in 4M NaOH (50 mL) and refluxed for 4 hours. The reaction mixture was cooled to room temperature and poured into 50 mL of water. The mixture was cooled in an ice bath and slowly brought to pH 3 with 6N HCl. The precipitate which formed was collected by filtration, washed with water and dried under vacuum to give an off-white solid (3.5 g, 66%).

1H NMR (DMSO-d6, 300 MHz): 12.03 (bs, 1H), 11.87 (s, 1H), 7.99-7.92 (m, 2H), 7.23 (dd, J=9.9 & 2.4 Hz, 1H),7.03-6.97 (m, 1H).

### (1R,3R,5S)-8-methyl-8-azabicyclo[3.2.1]octan-3-yl 6-fluoro-1H-indole-3-carboxylate (3)

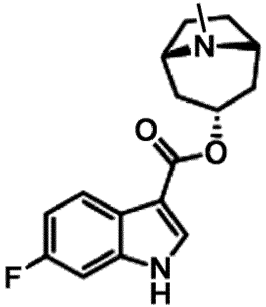

DMF (0.1 mL) was added to a stirring mixture of 6-fluoroiindole-3-carboxylic acid (3.500 g, 19.5 mmol, 1.0equiv.) and oxalyl chloride (3.306 ml, 39.1 mmol, 2.0 equiv.) in dichloromethane (40 mL) under nitrogen at 0_{∘}C(ice bath tmeperature). After 2 hours the reaction was concentrated to dryness. The residue was taken up in DCM (20 mL) and cooled to 0°C and tropine (8.276 g, 58.6 mmol, 3.0 equiv.) was added as a solution in DCM (25mL) with stirring. The reaction mixture was allowed to come to room temperature and stirred overnight. The reaction mixture was washed with saturated sodium bicarbonate and the solvents removed under reduced pressure. The residue was titurated with ethyl acetate and hexanes to get the product as a tan colored solid (2.34g, 39.6%).

1HNMR (DMSO-d6, 300 MHz): 11.94 (s, 1H), 8.01-7.96 (m, 2H), 7.27 (dd, J=9.9 & 2.4 Hz, 1H), 7.08-7.01 (m, 1H),5.06 (t, J=5.4 Hz, 1H), 3.09 (s, 2H), 2.21-1.99 (m, 9H), 1.75-1.70 (m, 2H). LC/MS: 303.6 (M+1).

### (1R,3R,SS)-8-methyl-8-azabicyclo[3.2.1]octan-3-yl 6-fluoro-1H-indole-3-carboxylate hydrochloride (4)

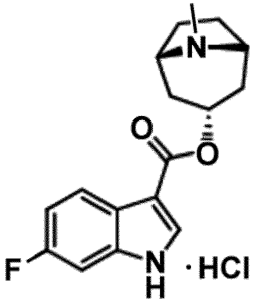

A round bottom flask was charged with (1R,3r,5S)-8-methyl-8-azabicyclo[3.2.1]octan-3-yl 6-fluoro-1H-indole-3-carboxylate (2.180 g, 7.2 mmol, 1.0 equiv.), 1,4-dioxan (20 mL) and HCl (4M in 1,4-dioxan)(1.803 ml, 7.2 mmol, 1.0 equiv.) was added under nitrogen. The reaction mixture was stirred at room temperature for 2 hours. The solvents were removed under reduced pressure and washed with ether two times. The residue was dried under vacuum in an oven to get the product as off white solid (2.30 g, 93.2%).

1H NMR (CD3OD, 300 MHz): 8.08-8.03 (m, 1H), 7.99 (s, 1H), 7.19 (dd, J=9.9 &2.4 Hz, 1H), 7.03-6.96 (m, 1H), 5.27(t, J=4.8 Hz, 1H), 3.94 (bs, 2H), 2.84 (s, 3H), 2.53-2.26 (m, 8H). LC/MS: 303.6 (M+1). HPLC 99% purity.

### Example 2

### Identification and Characterization of C41

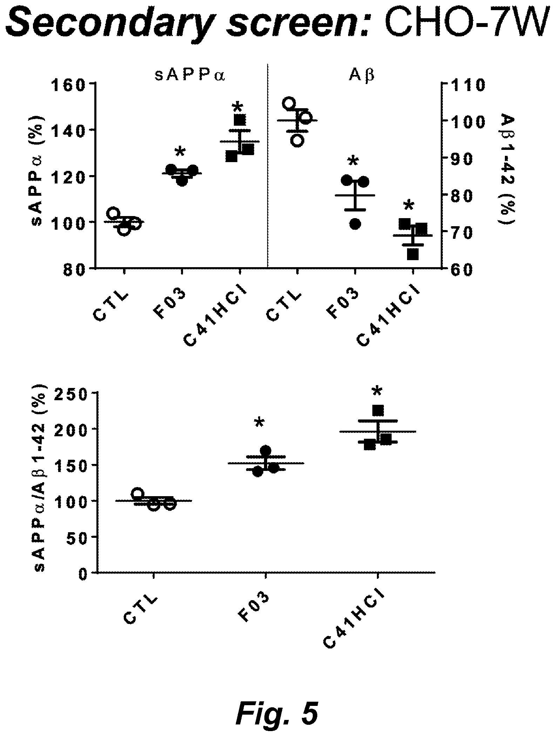

Compound C41 was initially identified by screening a library of compounds in a series of steps. First compounds were screened in a primary screen to determine the effect on sAPPα in SH-SY5Y cells (*see, e.g.,* Fig. 4). Compounds that elicited an increase in sAPPα of at least 10% were subjected to a secondary screen in CHO-7W cells for their effect on sAPPα and Aβ (*see, e.g.,* Fig. 5). Results of these screens are also shown in Table 4.

Compound C41 differs from tropisetron (also known as Navoban and F03) in the substitution of F in the 6-indole position (*see,* Figure 1 compared to Figure 3). Accordingly compound C41 was evaluated in comparison to tropisetron (F03).

**Table 4. Target binding and results of primary and secondary screen of C41 compared to F03 (tropisetron).**

| Cmpd. | Target | | | Primary Screen (SH-SY5Y) | Secondary Screen (CHO-7W) | | |
|---|---|---|---|---|---|---|---|
| | α7 (nM) | 5-HT3 (nM) | APP (µM) | sAPPα | sAPPα | Aβ1-42 | sAppα/Aβ |
| F03 | 450 | 3 | 1-10 | ↑>10% | ↑>10% | ↓ >10% | ↑>30% |
| C41 | 1100 | 5 | + | ↑∼20% | ↑>20% | ↓ >20% | ↑>50% |

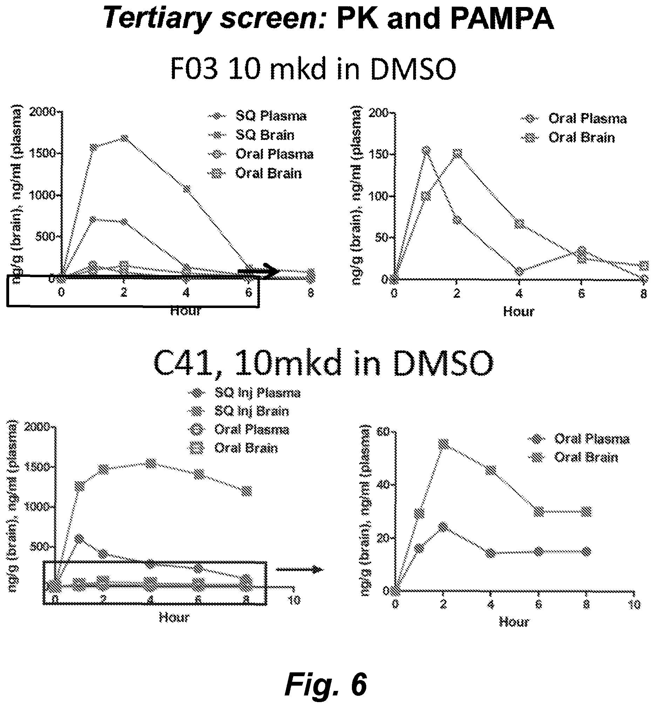

Compounds that showed an increase in sAPPα/Aβ ratio of at least 10 percent were screened in tertiary screen for brain permeability and pharmacokinetic (PK) parameters such as clearance (*see, e.g.*, Fig. 6, and Table 5). Compounds that showed a K_{IAM} greater than 0.6 and a brain/plasma ratio greater than or equal to 0.2 were subjected to various absorption, distribution, metabolism, excretion properties and toxicity (ADME-tox).

**Table 5. Results of tertiary screen for C41 and tropisetron (F03).**

| | Tertiary Screen | |
|---|---|---|
| Compound | PAMPA (K_{IAM}) | Solubility (In Water) |
| F03 | 4.04 | 100 mM |
| C41 | 3.57 | 85 mM |
| K(IAM) > 0.85 preferred for increased brain permeability. | | |

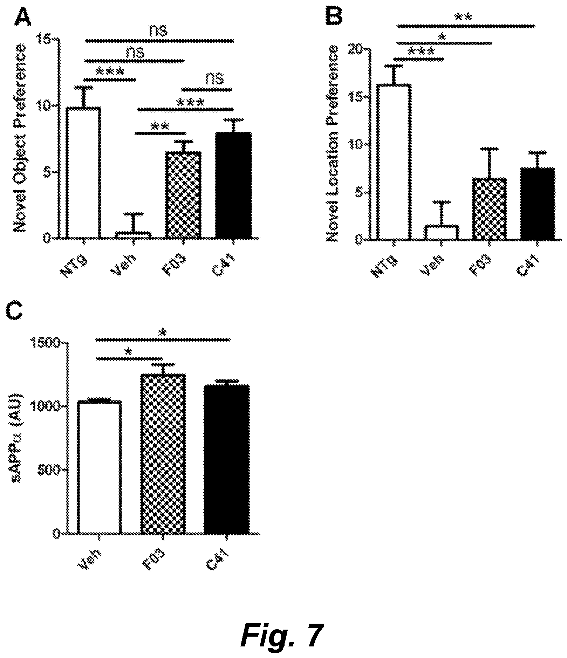

Compounds meeting the initial screening criteria were then subjected to *in vivo* testing. *In vivo,* compound C41 performed better than F03. In particular short-term working object memory improved significantly (t-test) in an AD model to approximately 85% of NTg level, which was greater improvement than that obtained with F03 (*see, e.g.,* Fig. 7, panel A). C41 also improved spatial memory as shown by a novel location recognition test (Fig. 7, panel B). Additionally, sAPPα increased significantly *in vivo* with F03 and C41 treatment (Fig. 7, panel C).

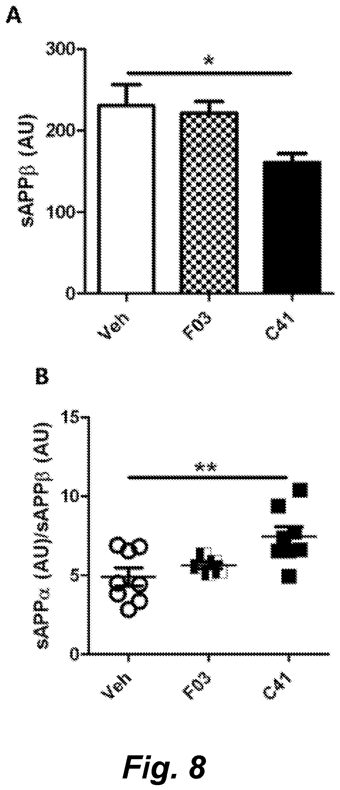

C41 also showed greater decrease in sAPPβ than F03 (Fig. 8, panel A), and the sAPPα/sAPPβ showed a significantly greater increase with C41 than F03 (Fig. 8, panel B).

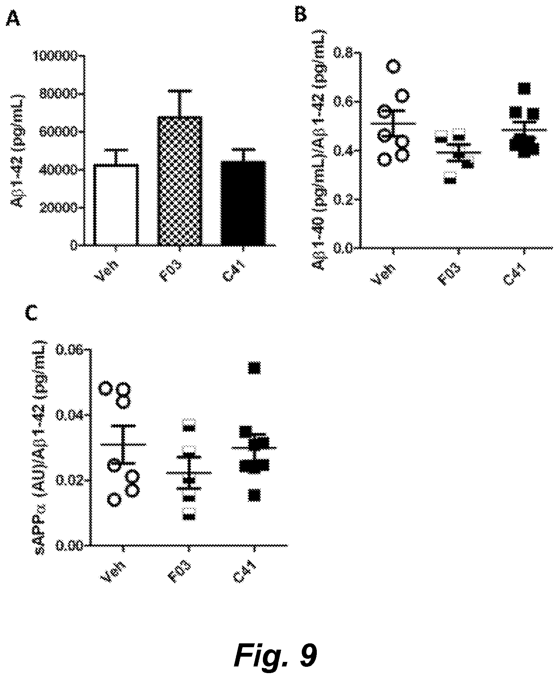

C41 did not change Aβ1-42 levels (Fig. 9, panel A). The Aβ40/42 ratio was not changed by either C41 or F03 (Fig. 9, panel B). Additionally the sAPPα/Aβ1-42 ratio was not increased with either compound (Fig. 9, panel C).

C41 showed a clean profile in a panel binding test. In particular, it showed binding to α7 and 5HT3 receptors (*see* Table 4), no binding to hERG receptor at 20× plasma concentration and only weak binding to muscarinic acetylcholine receptors.

Thus, C41 was identified as a good new chemical entity (NCE) based on the testing. In particular C41 *in vitro* and *in vivo* profile met the advancement criteria in SH-SY5Y and CHO-7W *in vitro* screens. C41 showed good brain permeability. Efficacy was seen in a 4-week standard oral study in mice. Efficacy was seen in dose-response studies in mice, and efficacy was seen in a 12-week study in old AD model mice with advanced pathology.

### Example 3

### Testing of a Second Batch of C41.

A second batch of C41 HCl was synthesized as described in Example 1. The second batch of C41 was tested for plasma protein binding, ADME-tox screening (microsomal intrinsic clearance), and ADME-tox screening (CYP inhibition screen). PK in rats was determined to determine a dose of non-GLP Pharm-Tox screening. Additionally, acute toxicity in rats (pre-subchronic toxicity) and non-GLP subchronic toxicity was determined.

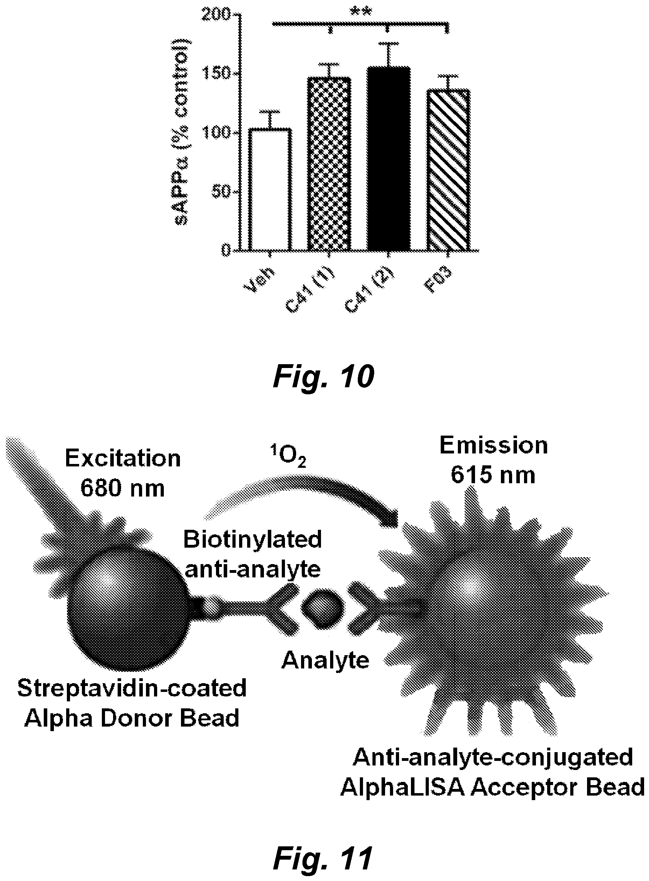

The second batch of C41 (C41(2) was shown to increase sAPPα similar to C41 (first batch) and to F03 in human neuroblastoma SH-SY5Y cells after exposure to 1 µm of each for 24 hours (*see, e.g.,* Fig. 10). The Perkin-Elmer AlphaLISA assay was used for sAPPα, sAPPβ, tau, and p-tau determinations. This assay is a bead-based assay where the analyte is bound by specific antibodies conjugated to the acceptor beads. Donor beads to which different analyte-specific antibodies are conjugated are added. The donor beads release singlet oxygen, and, due to close proximity to the acceptor bead, light is emitted.

The mean plasma fraction bound by C41 was lower than that of controls propranolol and warfarin (Table 6). Plasma binding for F03 has been reported to be 70%.

**Table 6. Plasma fraction bound by C41.**

| Test Article | Test Species | Mean Fraction Unbound Fuₚₗₐₛₘₐ (%) | Mean Plasma Fraction Bound | Post-Assay Recovery | Comment |
|---|---|---|---|---|---|
| propranolol | Human | 18.4% | 81.6% | 96% | binding control |
| warfarin | Human | 0.39% | 99.6% | 99.7% | binding control |
| C41 | Human | 25.7% | 74.3% | 115% | |

NADPH-dependent intrinsic clearance (Clint) of F03 and C41 were similar. Intermediate V:W values were similar in all cases except clearance in rat which was lower for C41. T_{1/2} for C41 in all species for NADPH-dependent clearance was >V (verapamil), < W (warfarin). C41 had a longer T_{1/2} than F03 as expected due to its CYP resistance *(see* Table 7).

**Table 7. C41 ADME-tox results.**

| Test Article | Test Conc. | Species | NADPH-Dependent CLint (µL min⁻¹ m_{g}⁻¹) | NADPH-Dependent T_{1/2} (Min) | NADPH-Free CLint (µL min⁻¹ mg⁻¹) | NADPH -Free T_{1/2} (Min) | Comment |
|---|---|---|---|---|---|---|---|
| F03 | 1 µM | Dog | 43.5 | 53.1 | <9.6 | >240 | |
| | | Human | <9.6 | >240 | <9.6 | >240 | 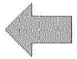 |
| | | Rat | 367 | 6.3 | <9.6 | >240 | |
| C41 | 1 µM | Dog | 45.7 | 50.6 | <9.6 | >240 | |
| | | Human | <9.6 | >240 | <9.6 | >240 | 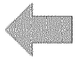 |
| | | Rat | 187 | 12.4 | <9.6 | >240 | |
| verapam il | 1 µM | Dog | 165 | 14.0 | <9.6 | >240 | high clearance control |
| | | Human | 212 | 10.9 | <9.6 | >240 | |
| | | Rat | 500 | 4.6 | <9.6 | >240 | |
| warfarin | 1 µM | Dog | <9.6 | >240 | <9.6 | >240 | low clearance control |
| | | Human | <9.6 | >240 | <9.6 | >240 | |
| | | Rat | <9.6 | >240 | <9.6 | >240 | |

In a CYP inhibition screen, as expected, C41 induced lower inhibition of CYP enzymes as compared to F03 *(see* Table 8).

**Table 8. CYP inhibition data.**

| Test Article | Test Conc. | CYP3A4 midazolam | CYP3A4 testosterone | CYP2C9 | CYP2D6 | CYP2C19 | CYP1A2 |
|---|---|---|---|---|---|---|---|
| F03 | 30 µM | 6.6% | 10.5% | 23.3% | 38.0% | 0.0% | 1.0% |
| | 10 µM | 0.0% | 0.63% | 15.9% | 17.4% | 6.4% | 1.0% |
| C41 | 30 µM | 3.8% | 2.5% | 21.6% | 36.1% | 0.0% | 0.33% |
| | 10 µM | 3.1% | 5.2% | 11.4% | 19.9% | 0.0% | 0.35% |

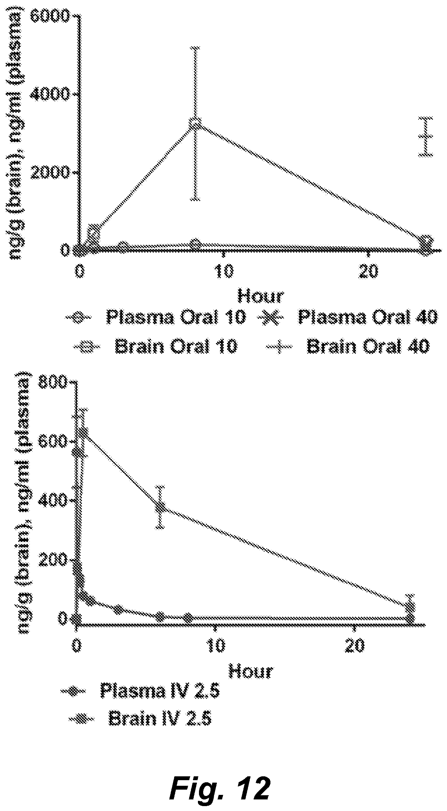

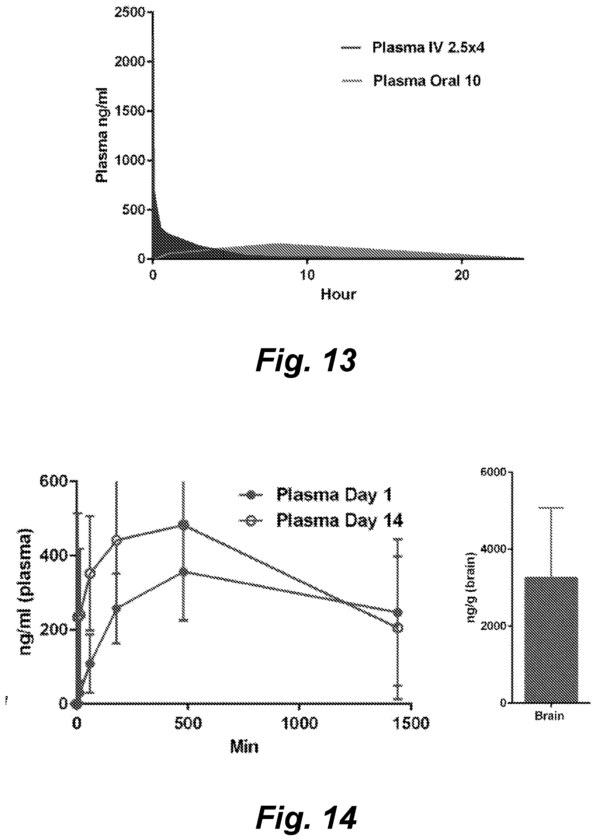

C41 pharmacokinetics were determined in a rat *(see,* Fig. 12, Table 9). The top panel of Fig. 12 shows the results of oral deliver at 10 mk. Brain tissue was collected at 1, 8, and 24 hours. Plasma peak was 150 ng/ml and brain was 3251 ng/g. C41 peaked at 8 hours. The bottom panel of Fig. 12, shows the results of intravenous delivery at 2.5 mk. Brain tissue was collected at 30 min, 6 hours, and 24 hours. Plasma peak was 564 ng/ml, at 1 minute and brain peak was 629 ng/g at 30 minutes. The 30 minute brain:plasma ratio was 8. Oral availability was determined to be about 100% (*see, e.g.* Fig. 13).

**Table 9. C41 pharmacokinetic parameters.**

| **Oral, plasma** | **IV, plasma** |
|---|---|
| Tₘₐₓ oral = 480 min. | Tₘₐₓ = 1.0 min. |
| T_{1/2} elimination = 154.022 min. | T_{1/2} elimination = 896.518 min. |
| Mean residence time (MRT) = 430.4 min. | Mean residence time (MRT) area min = 499.6 |
| Clearance (CL) ml/min/Kg = 0.084. | Clearance (CL) ml/min/Kg = 0.112 |
| Volume of distribution steady state ml/kg = 18.8. | Volume of distribution steady state ml/kg = 144.5. |
| Cₘₐₓ plasma = 150.2 ng/ml. | Cₘₐₓ plasma = 564.0 ng/ml. |
| AUC ng-min/ml = 118478.2 | AUC ng-min/ml = 22379.9. |
| AUC (0-t) ng-min/ml = 118033.7. | AUC (0-t) ng-min/ml = 19792.5. |

| **Oral, brain** | **IV brain** |
|---|---|
| Tₘₐₓ min = 380.0 | Tₘₐₓ min = 30.0 |
| T_{1/2} elimination min = 144.681. | T_{1/2} elimination min = 333.564. |
| Mean residence time (MRT) min = 1303.8. | Mean residence time (MRT) min = 398.2. |
| Clearance (CL) g/min/Kg = 0.087. | Clearance (CL) g/min/Kg = 0.006. |
| Volume of distribution steady state g/Kg = -18.2. | Volume of distribution steady state g/Kg = 2.9. |
| Cₘₐₓ ng/g = 3251.0 | Cₘₐₓ ng/g = 629.0 |
| AUC ng-min/g = 114944.7 | AUC ng-min/g = 419858.3 |
| AUC (0-t) ng-min/g = 793672.5 | AUC (0-t) ng-min/g = 400605.0 |

Figure 14 shows subchronic non-GLP toxicity of C41 after oral gavage in rats for 14 days, done at Absorption Systems. No weight loss and no adverse effects were observed.

Acute non-GLP toxicity study was performed using an oral dose of 450 mg/kg. At 24 hours post-dosing, brain levels were 2924 ng/g and plasma levels were 115 ng/ml. There was some dilation of the vessels on the surface of the intestine which was likely due to the corn syrup vehicle as references indicated that this vehicle elicits this response.

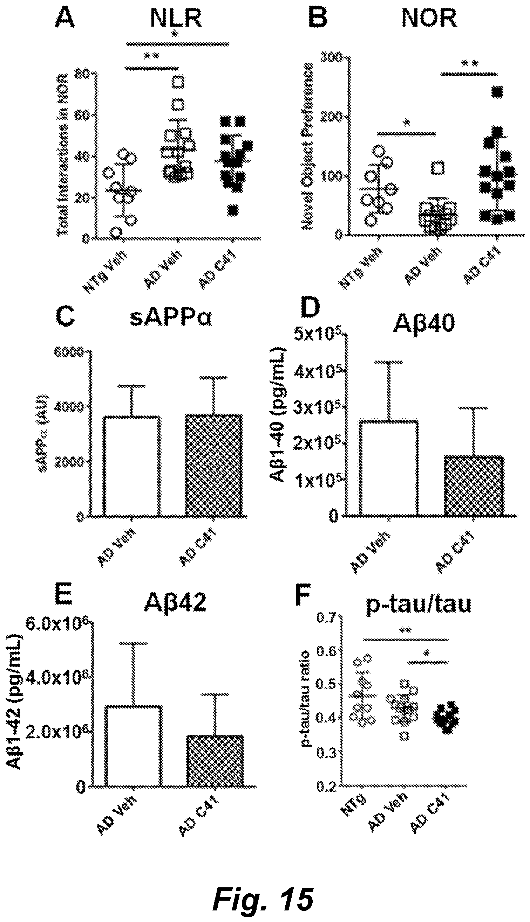

There was some improvement in cognition in novel location recognition (Figure 15, panel A) and significant improvement in novel object recognition (Figure 15, panel B) tasks after 4 weeks or oral dosing of B254 mice at 4 mkd, there was a trend for Aβ40 and 42 to decrease, and the p-Tau/Tau ratio significantly decreased (Figure 15, panels C-F).

Mechanistic confirmation studies for determination of synaptic load were performed using antibody-labeling of synaptophysin with a green fluorescent tag. Both F03 and C41 restored synaptic load in hippocampus (*see, e.g.,* Fig. 15). The improvements in NLR and NOR may be correlated to the restoration of synaptic load, increases in sAPPα or the sAPPα/sAPPβ ratio; increases in the sAPPα/Aβ1-42 ratio; decreases in the p-tau/tau ratio, and/or decreases in APPneo (c-terminal cleavage).

### Example 2

### C41 Characterization

Table 10 shows C41 stability in the S9 liver microsome of six species including human.

**Table 10. C41 S9 stability.**

| | Metabolic Half-Life (min) | | | | | |
|---|---|---|---|---|---|---|
| Test Article | Human S9 | Monkey S9 | Dog S9 | Rat S9 | Mouse S9 | Guinea Pig S9 |
| NTW-2134 (Positive Control) | 7.40 | 5.48 | | 5.75 | 10.53 | 5.60 |
| NTW-3456 (Positive Control) | | | 22.0 | | | |
| C41 | >60 | >60 | 40.1 | 21.8 | >60 | >60 |

C41 is stable in the S9 liver microsome of all six species, especially in human, monkey, mouse, and guinea pig with T_{1/2} >60 mins. Faster metabolism was observed in rat and dog.

Table 11 illustrates results of an *in silico* StarDRop analysis. As shown therein, StarDrop predicts brain permeability.

**Table 11. Oral CNS scoring profile comparing C41 and F03 obtained using the StarDrop software ver 6.3 (Optibriium Ltd).**

| Compound Name Structure MW | Oral CNS Scoring Profile | TPSA | logP | BBB log([brain]:[blood]) BBB category |
|---|---|---|---|---|
| 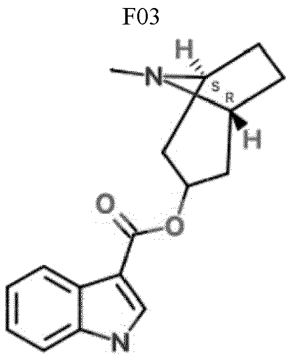 | 0.1515 | 45.33 | 3.866 | 0.764 + |
| 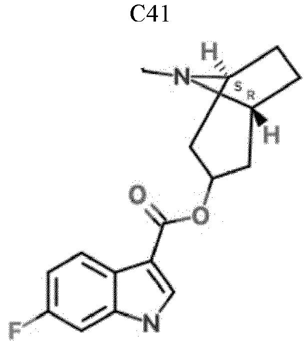 | 0.1148 | 45.33 | 3.954 | 0.8179 + |

Table illustrates a profile of various properties of C41.

**Table 12. Compound profile for C41.**

| **Category** | **Parameter** | **Activity/Value** | **Comments** |
|---|---|---|---|
| Chemistry | Molecular weight | 303.34 | |
| | Solubility | 80 mM | |
| | cLogP | 2.86 | |
| | SAR | Focused F03 analogs | |
| Pharmacology | Primary | 5HT3 antag. Kd ∼ 3nM | |
| | Secondary | | |
| | *in vitro* pharmacology -Activity in broad screening panel | Overall clean profile. Some binding to muscarinic receptors | Cerep panel |
| DMPK *In vitro* | In vitro permeability (PAMPA) | 3.6 | >0.9 predicts good brain permeability. F03 ∼ 4 |
| | Plasma protein binding | 74.3 (human) | |
| | Microsomal stability | T_{1/2} > 240 min (human) | Cyprotex |
| | P450 inhibition | >30µM CYP3A4, CYP2C9, CYP2D6 CYP2C19, CYP1A2 | Cyprotex |
| DMPK *In vivo* | Unbound brain to plasma | 22 | Rat |
| | Oral bioavailability (F) | ∼ 98% | Rat, IV % PO (2.5mg/kg & 10 mg/kg) |
| | AUC (ng.min/mL) | 22379 (IV) 118033 (PO) | Rat, IV % PO (2.5mg/kg & 10 mg/kg) |
| | MRT (min) | 398 (IV) 1304 (PO) | Rat, IV % PO (2.5mg/kg & 10 mg/kg) |
| Toxicology | hERG activity | No activity, >1µM | Cerep |
| | Non-GLP at 40 mg/kg | No adverse effects | Acute, Absorption Systems |

As shown therein C41 has high water solublity. C41 also demonstrates excellent brain to plasma ration and escellent oral bioavailability. No adverse effects were observed at high dosage.

## Claims

1. A compound according to the formula 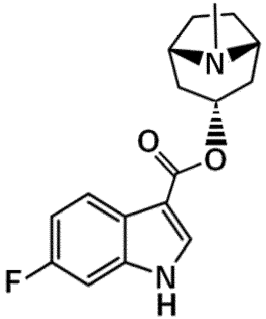 or an enantiomer, a mixture of enantiomers, or a mixture of two or more diastereomers thereof; or a pharmaceutically acceptable salt, , , solvate or hydrate thereof

2. The compound of claim 1, wherein said compound comprises a pharmaceutically acceptable salt.

3. The compound of claim 2, wherein said pharmaceutically acceptable salt is a salt of an acid selected from the group consisting of 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid (D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (- L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid (- L), salicylic acid, sebacic acid, stearic acid, succinic acid, sulfuric acid, tartaric acid (+ L), thiocyanic acid, toluenesulfonic acid (p), acid undecylenic acid.

4. The compound of claim 2, wherein said pharmaceutically acceptable salt is HCl.

5. A pharmaceutical formulation comprising the compound according to any one of claims 1-4, and a pharmaceutically acceptable carrier or excipient.

6. The formulation of claim 5, wherein said formulation is formulated for administration via a route selected from the group consisting of oral administration, nasal administration, administration via inhalation, oral administration, rectal administration, intraperitoneal injection, intravascular injection, subcutaneous injection, transcutaneous administration, and intramuscular injection.

7. The formulation according to any one of claims 5-6, wherein said formulation is a unit dosage formulation.

8. The formulation according to any one of claims 5-7, wherein said formulation is sterile.

9. A compound according to any one of claims 1-4, or a pharmaceutical formulation according to any one of claims 5-8, for use in a method of mitigating in a mammal one or more symptoms associated with a disease **characterized by** amyloid deposits in the brain, or delaying or preventing the onset of said symptoms, said method comprising:
administering, or causing to be administered, to said mammal said compound or formulation, wherein said administering is in an amount sufficient to mitigate said one or more symptoms.

10. A compound according to any one of claims 1-4, or a pharmaceutical formulation according to any one of claims 5-8, for use in a method of reducing the risk, lessening the severity, or delaying the progression or onset of a disease **characterized by** beta-amyloid (Aβ) deposits in the brain of a mammal, said method comprising:
administering, or causing to be administered, to said mammal said compound or formulation, wherein said administering is in an amount sufficient to reducing the risk, lessen the severity, or delay the progression or onset of said disease.

11. The compound or formulation for use according to claim 9 or 10, wherein said disease is a disease selected from the group consisting of Alzheimer's disease,
Cerebrovascular dementia, Parkinson's disease, Huntington's disease, Cerebral amyloid angiopathy (CAA), amytrophic lateral sclerosis (ALS), traumatic brain injury (TBI), and stroke.

12. A compound according to any one of claims 1-4, or a pharmaceutical formulation according to any one of claims 5-8, for use in a method of preventing or delaying the onset of a pre-Alzheimer's condition and/or cognitive dysfunction, and/or ameliorating one or more symptoms of a pre-Alzheimer's condition and/or cognitive dysfunction, or preventing or delaying the progression of a pre-Alzheimer's condition or cognitive dysfunction to Alzheimer's disease in a mammal, said method comprising:
administering, or causing to be administered, to said mammal said compound or formulation, wherein said administering is in an amount sufficient to promote the processing of amyloid precursor protein (APP) by the non-amyloidogenic pathway.

13. The compound or formulation for use according to any one of claims 9 to 12, wherein administration of said compound or formulation delays or prevents the progression of MCI to Alzheimer's disease.

14. The compound or formulation for use according to any one of claims 9-11, wherein the disease is Alzheimer's disease.

15. The compound or formulation for use according to any one of claims 9 to 14, wherein the mammal is at risk of developing Alzheimer's disease.

16. The compound or formulation for use according to claim 15, wherein the mammal has a familial risk for having Alzheimer's disease, and/or the mammal has a familial Alzheimer's disease (FAD) mutation, and/or the mammal has the APOE ε4 allele.

17. A kit comprising:
a container containing a compound according to any one of claims 1-4, or a pharmaceutical formulation according to any one of claims 5-8; and
instructional materials teaching the use of said composition to mitigate one or more symptoms associated with a disease **characterized by** amyloid deposits in the brain, and/or the use of said composition in delaying or preventing the onset of one or more of said symptoms.

## Patentansprüche

1. Verbindung gemäß der Formel 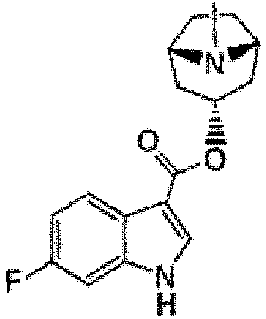 oder ein Enantiomer, ein Gemisch von Enantiomeren, oder ein Gemisch von zwei oder mehr Diastereomeren davon; oder ein pharmazeutisch annehmbares Salz, Solvat oder Hydrat davon.

2. Verbindung nach Anspruch 1, wobei die Verbindung ein pharmazeutisch annehmbares Salz umfasst.

3. Verbindung nach Anspruch 2, wobei das pharmazeutisch annehmbare Salz Folgendes ist: ein Salz einer Säure, ausgewählt aus der Gruppe bestehend aus 1-Hydroxy-2-naphthoesäure, 2,2-Dichloressigsäure, 2-Hydroxyethansulfonsäure, 2-Oxoglutarsäure, 4-Acetamidobenzoesäure, 4-Aminosalicylsäure, Essigsäure, Adipinsäure, Ascorbinsäure (L), Asparaginsäure (L), Benzolsulfonsäure, Benzoesäure, Camphersäure (+), Campher-10-Sulfonsäure (+), Caprinsäure (Decansäure), Capronsäure (Hexansäure), Caprylsäure (Octansäure), Kohlensäure, Zimtsäure, Zitronensäure, Cyclamsäure, Dodecylschwefelsäure, Ethan-1,2-disulfonsäure, Ethansulfonsäure, Ameisensäure, Fumarsäure, Galaktarsäure, Gentisinsäure, Glucoheptonsäure (D), Gluconsäure (D), Glucuronsäure (D), Glutaminsäure, Glutarsäure, Glycerophosphorsäure, Glykolsäure, Hippursäure, Bromwasserstoffsäure, Salzsäure, Isobuttersäure, Milchsäure (DL), Lactobionsäure, Laurinsäure, Maleinsäure, Apfelsäure (- L), Malonsäure, Mandelsäure (DL), Methansulfonsäure, Naphthalin-1,5-disulfonsäure, Naphthalin-2-sulfonsäure, Nikotinsäure, Salpetersäure, Ölsäure, Oxalsäure, Palmitinsäure, Pamoinsäure, Phosphorsäure, Proprionsäure, Pyroglutaminsäure (- L), Salicylsäure, Sebacinsäure, Stearinsäure, Bernsteinsäure, Schwefelsäure, Weinsäure (+ L), Thiocyansäure, Toluolsulfonsäure (p), Undecylensäure.

4. Verbindung nach Anspruch 2, wobei das pharmazeutisch annehmbare Salz HCl ist.

5. Pharmazeutische Formulierung, die die Verbindung nach einem der Ansprüche 1-4 und einen pharmazeutisch annehmbaren Träger oder Exzipienten umfasst.

6. Formulierung nach Anspruch 5, wobei die Formulierung zur Verabreichung über einen Weg formuliert ist, der ausgewählt ist aus der Gruppe bestehend aus oraler Verabreichung, nasaler Verabreichung, Verabreichung durch Inhalation, oraler Verabreichung, rektaler Verabreichung, intraperitonealer Injektion, intravaskulärer Injektion, subkutaner Injektion, transkutaner Verabreichung und intramuskulärer Injektion.

7. Formulierung nach einem der Ansprüche 5-6, wobei die Formulierung eine Einheitsdosierungsformulierung ist.

8. Formulierung nach einem der Ansprüche 5-7, wobei die Formulierung steril ist.

9. Präparat nach einem der Ansprüche 1-4, oder pharmazeutische Formulierung nach einem der Ansprüche 5-8, zur Verwendung in einem Verfahren zur Milderung eines oder mehrerer Symptome in einem Säuger, die einer Krankheit zuzuordnen sind, die durch Amyloid-Ablagerungen im Gehirn gekennzeichnet ist, oder zur Verzögerung oder Verhinderung des Auftretens der Symptome, wobei das Verfahren Folgendes umfasst:
Verabreichen oder Veranlassen der Verabreichung der Verbindung oder Formulierung an den Säuger, wobei die Verabreichung in einer Menge erfolgt, die ausreicht, um eines oder mehrere Symptome zu lindern.

10. Verbindung nach einem der Ansprüche 1-4, oder pharmazeutische Formulierung nach einem der Ansprüche 5-8, zur Verwendung in einem Verfahren zur Verringerung des Risikos, zur Milderung des Schweregrades oder zur Verzögerung des Fortschreitens oder Auftretens einer Krankheit, die durch Beta-Amyloid (Aβ)-Ablagerungen im Gehirn eines Säugers gekennzeichnet ist, wobei das Verfahren Folgendes umfasst:
Verabreichen oder Veranlassen der Verabreichung der Verbindung oder Formulierung an den Säuger, wobei die Verabreichung in einer ausreichenden Menge erfolgt, um das Risiko zu verringern, den Schweregrad zu verringern oder das Fortschreiten oder den Ausbruch der Krankheit zu verzögern.

11. Verbindung oder Formulierung zur Verwendung nach Anspruch 9 oder 10, wobei die Krankheit eine Krankheit ist, ausgewählt aus der Gruppe bestehend aus Alzheimer-Krankheit, zerebrovaskulärer Demenz, Parkinson-Krankheit, Huntington-Krankheit, zerebraler Amyloid-Angiopathie (Cerebral Amyloid Angiopathy, CAA), amytropher Lateralsklerose (ALS), traumatischer Hirnverletzung (Traumatic Brain Injury, TBI) und Schlaganfall.

12. Verbindung nach einem der Ansprüche 1-4, oder pharmazeutische Formulierung nach einem der Ansprüche 5-8, zur Verwendung in einem Verfahren zur Verhinderung oder Verzögerung des Auftretens einer Vor-Alzheimer-Krankheit und/oder kognitiver Dysfunktion, und/oder zur Linderung eines oder mehrerer Symptome einer Vor-Alzheimer-Krankheit und/oder kognitiven Dysfunktion, oder zur Verhinderung oder Verzögerung des Fortschreitens einer Vor-Alzheimer-Krankheit oder kognitiven Dysfunktion zur Alzheimer-Krankheit in einem Säuger, wobei das Verfahren Folgendes umfasst:
Verabreichen oder Veranlassen der Verabreichung der Verbindung oder Formulierung an den Säuger, wobei die Verabreichung in einer ausreichenden Menge erfolgt, um die Verarbeitung von Amyloid-Vorläuferprotein (Amyloid Precursor Protein, APP) durch den nicht-amyloidogenen Pfad zu fördern.

13. Verbindung oder Formulierung zur Verwendung nach einem der Ansprüche 9 bis 12, wobei die Verabreichung der Verbindung oder Formulierung das Fortschreiten von MCI zur Alzheimer-Krankheit verzögert oder verhindert.

14. Verbindung oder Formulierung zur Verwendung nach einem der Ansprüche 9-11, wobei die Krankheit die Alzheimer-Krankheit ist.

15. Verbindung oder Formulierung zur Verwendung nach einem der Ansprüche 9 bis 14, wobei der Säuger gefährdet ist, die Alzheimer-Krankheit zu entwickeln.

16. Verbindung oder Formulierung zur Verwendung nach Anspruch 15, wobei der Säuger ein familiäres Risiko hat, an Alzheimer zu erkranken, und/oder der Säuger eine familiäre Alzheimer-Mutation (FAD) hat, und/oder der Säuger das APOE ε4-Allel besitzt.

17. Kit, das Folgendes umfasst:
einen Behälter, der eine Verbindung nach einem der Ansprüche 1-4 enthält, oder eine pharmazeutische Formulierung nach einem der Ansprüche 5-8; und
Anleitungsmaterialien, die die Verwendung der Zusammensetzung zur Linderung eines oder mehrerer Symptome die einer Krankheit zuzuordnen sind, die durch Amyloidablagerungen im Gehirn gekennzeichnet ist, und/oder die Verwendung der Zusammensetzung zur Verzögerung oder Verhinderung des Auftretens eines oder mehrerer der Symptome lehren.

## Revendications

1. Composé, selon la formule 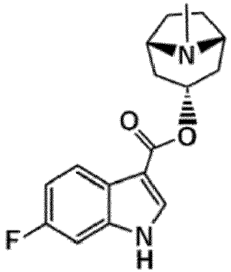 ou un énantiomère, un mélange d'énantiomères, ou un mélange de deux, ou plus, diastéréomères de celui-ci ; ou un sel pharmaceutiquement acceptable, solvate ou hydrate de celui-ci.

2. Composé selon la revendication 1, dans lequel ledit composé comprend un sel pharmaceutiquement acceptable.

3. Composé selon la revendication 2, dans lequel ledit sel pharmaceutiquement acceptable est un sel d'un acide sélectionné parmi le groupe constitué de : acide 1-hydroxy-2-naphtoïque, acide 2,2-dichloroacétique, acide 2-hydroxyéthanesulfonique, acide 2-oxoglutarique, acide 4-acétamidobenzoïque, acide 4-aminosalicylique, acide acétique, acide adipique, acide ascorbique (L), acide aspartique (L), acide benzènesulfonique, acide benzoïque, acide camphorique (+), acide camphor-10-sulfonique (+), acide caprique (acide décanoïque), acide caproïque (acide hexanoïque), acide caprylique (acide octanoïque), acide carbonique, acide cinnamique, acide citrique, acide cyclamique, acide dodécylsulfurique, acide éthane-1,2-disulfonique, acide éthanesulfonique, acide formique, acide fumarique, acide galactarique, acide gentisique, acide glucoheptonique (D), acide gluconique (D), acide glucuronique (D), acide glutamique, acide glutarique, acide glycérophosphorique, acide glycolique, acide hippurique, acide hydrobromique, acide hydrochlorique, acide isobutyrique, acide lactique (DL), acide lactobionique, acide laurique, acide maléique, acide malique (- L), acide malonique, acide mandélique (DL), acide méthanesulfonique, acide naphtalène-1,5-disulfonique, acide naphtalène-2-sulfonique, acide nicotinique, acide nitrique, acide oléique, acide oxalique, acide palmitique, acide pamoïque, acide phosphorique, acide proprionique, acide pyroglutamique (- L), acide salicylique, acide sébacique, acide stéarique, acide succinique, acide sulfurique, acide tartarique (+ L), acide thiocyanique, acide toluènesulfonique (p), acide undécylénique.

4. Composé selon la revendication 2, dans lequel ledit sel pharmaceutiquement acceptable est HCl.

5. Préparation pharmaceutique, comprenant le composé selon l'une quelconque des revendications 1 à 4, et un vecteur ou excipient pharmaceutiquement acceptable.

6. Préparation selon la revendication 5, dans laquelle ladite préparation est préparée pour l'administration par l'intermédiaire d'une voie sélectionnée parmi le groupe constitué de : administration orale, administration nasale, administration par inhalation, administration orale, administration rectale, injection intrapéritonéale, injection intravasculaire, injection sous-cutanée, administration transcutanée, et injection intramusculaire.

7. Préparation selon l'une quelconque des revendications 5 et 6, dans laquelle ladite préparation est une préparation à dosage unitaire.

8. Préparation selon l'une quelconque des revendications 5 à 7, dans laquelle ladite préparation est stérile.

9. Composé selon l'une quelconque des revendications 1 à 4, ou préparation pharmaceutique selon l'une quelconque des revendications 5 à 8, pour l'utilisation dans un procédé de mitigation, chez un mammifère, d'un ou de plusieurs symptômes associés à une maladie **caractérisée par** des dépôts amyloïdes dans le cerveau, ou de retardement ou de prévention du début desdits symptômes, ledit procédé comprenant :
l'administration, ou l'administration provoquée, audit mammifère, dudit composé ou de ladite préparation, dans lequel ladite administration est en une quantité suffisante pour mitiger ledit un ou lesdits plusieurs symptômes.

10. Composé selon l'une quelconque des revendications 1 à 4, ou préparation pharmaceutique selon l'une quelconque des revendications 5 à 8, pour l'utilisation dans un procédé de réduction du risque, d'amoindrissement de la sévérité, ou du retardement de la progression ou du début d'une maladie **caractérisée par** des dépôts bêta-amyloïdes (Aβ) dans le cerveau d'un mammifère, ledit procédé comprenant :
administration, ou l'administration provoquée, audit mammifère, dudit composé ou de ladite préparation, dans lequel ladite administration est en une quantité suffisante pour réduire le risque, amoindrir la sévérité, ou retarder la progression ou le début de ladite maladie.

11. Composé ou préparation pour l'utilisation selon la revendication 9 ou 10, dans lequel ou laquelle ladite maladie est une maladie sélectionnée parmi le groupe constitué de : maladie d'Alzheimer, démence cérébrovasculaire, maladie de Parkinson, maladie de Huntington, angiopathie amyloïde cérébrale (AAC), sclérose latérale amyotrophique (SLA), lésion cérébrale traumatique (LCT), et accident vasculaire cérébral.

12. Composé selon l'une quelconque des revendications 1 à 4, ou préparation pharmaceutique selon l'une quelconque des revendications 5 à 8, pour l'utilisation dans un procédé de prévention ou de retardement du début d'une condition pré-Alzheimer et/ou d'un dysfonctionnement cognitif, et/ou d'amélioration d'un ou de plusieurs symptômes d'une condition pré-Alzheimer et/ou d'un dysfonctionnement cognitif, ou de prévention ou de retardement de la progression d'une condition pré-Alzheimer ou d'un dysfonctionnement cognitif relatif à la maladie d'Alzheimer chez un mammifère, ledit procédé comprenant :
l'administration, ou l'administration provoquée, audit mammifère, dudit composé ou de la préparation, dans lequel ou laquelle ladite administration est en une quantité suffisante pour favoriser le traitement de protéine précurseur de l'amyloïde (APP) par le biais de la voie non amyloïdogénique.

13. Composé ou préparation pour l'utilisation selon l'une quelconque des revendications 9 à 12, dans lequel ou laquelle l'administration dudit composé ou de ladite préparation cause le retardement ou la prévention de la progression de TCL relatif à la maladie d'Alzheimer.

14. Composé ou préparation pour l'utilisation selon l'une quelconque des revendications 9 à 11, dans lequel ou laquelle la maladie est la maladie d'Alzheimer.

15. Composé ou préparation pour l'utilisation selon l'une quelconque des revendications 9 à 14, dans lequel ou laquelle le mammifère est à risque de développer la maladie d'Alzheimer.

16. Composé ou préparation pour l'utilisation selon la revendication 15, dans lequel ou laquelle le mammifère présente un risque familial d'être atteint de maladie d'Alzheimer, et/ou le mammifère présente une mutation de maladie d'Alzheimer familiale (MAf), et/ou le mammifère présente l'allèle ε4 d'APOE.

17. Kit, comprenant :
un récipient contenant un composé selon l'une quelconque des revendications 1 à 4, ou une préparation pharmaceutique selon l'une quelconque des revendications 5 à 8 ; et
des instructions expliquant l'utilisation de ladite composition pour mitiger un ou plusieurs symptômes associés à une maladie **caractérisée par** des dépôts amyloïdes dans le cerveau, et/ou l'utilisation de ladite composition dans le retardement ou la prévention du début d'un ou de plusieurs desdits symptômes.
